# EUROPEAN PATENT APPLICATION

(11) **EP 2 357 000 A1**
(43) Date of publication of application: **17.08.2011**
(21) Application number: 11156460.5
(22) Date of filing: 17.10.2006
(51) Int. Cl.: A61K 39/00, A61K 39/295, C12N 15/88

(54) **Mucosal and systemic immunizations with alphavirus replicon particles**

(30) Priority: 18.10.2005 US 727731 P
(62) Divisional of application: 06826178.3
(71) Applicant: Novartis Vaccines and Diagnostics, Inc., Emeryville, CA 94608 (US)
(72) Inventor: Vajdy, Michael, Emeryville, CA 94662-8097 (US)
(74) Representative: Hallybone, Huw George

(57) **Abstract**

Mucosal and systemic administration of compositions comprising alphavirus replicon particles to induce immune responses in a subject is described.

## Description

### RELATED APPLICATIONS

This application claims the benefit of U.S. Provisional Application No. 60/727,731, filed October 18, 2005, which teachings are incorporated herein in their entirety by reference.

### GOVERNMENT SUPPORT

The invention was supported, in whole or in part, by R21 grant 1 R21 AI50430-01 1 and IPCAVD grant 1 U19 AI51596 from the National Institutes of Health. The Government has certain rights in the invention.

### TECHNICAL FIELD _

The present invention relates generally to methods of modulating or generating an immune response using mucosal and/or systemic immunization techniques. In particular, the invention relates to methods of modulating or generating an immune response at mucosal and/or systemic compartments by mucosally and/or systemically administering alphavirus replicon particles to a subject.

### BACKGROUND OF THE INVENTION

Alphavirus replicon vectors are currently used as a vector platform to develop vaccines to control infectious disease. Such alphavirus replicon vectors have been derived from alphaviruses, which are enveloped, positive-stranded RNA viruses comprising a genus of genetically, structurally, and serologically related arthropod-borne viruses of the *Togaviridae* family. Twenty-six known viruses and virus subtypes have been classified within the alphavirus genus, including, Semliki Forest virus (SFV), Ross River virus (RRV), Venezuelan equine encephalitis virus (VEE) and Sindbis virus (SIN), the prototype member of the genus. Alphavirus members currently being developed into replicon vectors for vaccine applications include, for example, Semliki Forest virus (Liljestrom (1991) Bio/Technology 9:1356-1361; Berglund et al. (1998) Nat. Biotech. 16:562-565*),* Venezuelan equine encephalitis virus (Pushko et al. (1997) Virology 239:389-401), Sindbis virus (Xiong et al. (1989) Science 243:1188-1191; Dubensky et al. (1996) J. Virol. 70:508-519; Hariharan et al. (1998) J. Virol. 72:950-958; Polo et al. (1999) Proc. Natl. Acad. Sci. USA 96:4598-4603), and combinations thereof, including for example VEE-derived replicon RNA and SIN-derived surface glycoproteins (VEE/SIN) (Perri et al. (2003) J. Virol. 77:10394-10403). Alphavirus-based replicon vectors are devoid of the viral structural protein genes, but maintain the replication elements necessary for cytoplasmic RNA self-amplification and expression of the inserted heterologous gene(s) via an alphaviral RNA promoter. The absence of structural protein genes ensures that the replicons are completely defective and incapable of producing infectious virus.

Delivery strategies for alphavirus and other replicon vectors have focused primarily on packaging of the RNA vector into replication-defective virus-like particles, thus exploiting the natural receptor-mediated entry process, similar to viral infection. Replicon particles harboring the RNA vector also exhibit tropism for particular *in vivo* cell types based on the parental virus source of the structural coat proteins used for packaging, thus enabling exploitation of desirable properties such as mucosal delivery and the *in vivo* targeting of dendritic cells. As alphavirus replicon vectors do not encode the viral structural proteins necessary for packaging, production of replicon particles is achieved by providing the structural proteins in *trans,* in suitable cultured cells. Typically, the necessary complement of alphavirus structural proteins is provided either by the transient cotransfection of *in vitro* transcribed replicon and helper RNA encoding the structural proteins, or by introducing the replicons into packaging cell lines (PCL) that express the structural proteins from one or more DNA expression cassettes. Production of replicon particles in this manner preserves the replication-defective nature of the vectors, as the genetic information for the structural proteins remains absent.

The alphavirus replicon particle strategy for RNA vaccines has been evaluated using many diverse antigens, in a variety of animal models. Alphavirus replicon particles have been shown to induce cellular, humoral and mucosal immune responses following immunization. For example, mucosal administration of alphavirus replicon particles expressing human immunodeficiency virus (HIV) antigens has been shown to induce immune responses to the HIV antigens (Vajdy et al. (2001) 1 Infect. Dis. 184:1613-1616; Gupta et al. (2005) J. Virol. 79:7135-7145)*.* Systemic or mucosal immunization with alphavirus particles expressing respiratory syncytial virus (RSV) antigens has also been shown to induce an immune response to the RSV antigens.

Despite these results, there remains a need for the development of vaccines and vaccination strategies for obtaining improved immune responses to pathogens and controlling infectious disease. There remains a need for the development of vaccines and vaccination strategies for eliciting, inducing, stimulating, enhancing or boosting immune responses in mammals to various pathogens or cancers for which there are currently few or no effective vaccines and/or treatments.

### SUMMARY OF THE INVENTION

The present invention provides methods for inducing or generating an immune response in a mammal against one or more pathogen (e.g., bacteria, virus, tumor, etc.). The present invention provides methods for inducing or generating an immune response in a mammal against at least one antigen. In one aspect of the invention, the method comprises (i) administering mucosally to a mammal a first composition which comprises a first virus replicon particle and (ii) administering systemically to the mammal a second composition which comprises a second virus replicon particle, in either order. The virus replicon particles encode one or more (at least one) antigen of interest (target antigen). Virus replicon particles include alphavirus replicon particles, adenovirus replicon particles and poxvirus replicon particles. In a particular embodiment, the method comprises (i) administering mucosally to a mammal a first composition which comprises a first alphavirus replicon particle and (ii) administering systemically to the mammal a second composition which comprises a second alphavirus replicon particle, in either order. The first and second virus replicon particles are ' capable of expressing at least one target antigen. In a particular embodiment, the first and second virus replicon particles are capable of expressing the same target antigen or antigens. In another embodiment, the first and second virus replicon particles are capable of expressing at least one different target antigen.

In another aspect of the invention, the method comprises (i) administering mucosally to a mammal a first composition which comprises an alphavirus replicon particle and (ii) administering systemically to the mammal a second composition which comprises a non-alphavirus replicon particle, in either order. In a third aspect of the invention, the method comprises (i) administering mucosally to a mammal a first composition which comprises a non-alphavirus replicon particle and (ii) administering systemically to the mammal a second composition which comprises an alphavirus replicon particle, in either order. As above, in each of these methods, the virus replicon particles encode one or more (at least one) target antigen.

In a fourth aspect of the invention, the method comprises (i) administering to a mammal via a mucosal route of administration an effective amount of a priming composition which comprises a first virus replicon particle and (ii) subsequently administering to the mammal via a systemic route of administration an effective amount of a boosting composition which comprises a second virus replicon particle. In another aspect of the invention, the method comprises (i) administering to a mammal via a systemic route of administration an effective amount of a priming composition which comprises a first virus replicon particle and (ii) subsequently administering to the mammal via a mucosal route of administration an effective amount of a boosting composition which comprises a second virus replicon particle. The virus replicon particles encode one or more (at least one) antigen of interest (target antigen). The first and second virus replicon particles can be the same or different viral replicon particles. In a particular embodiment, the first and second virus replicon particles are alphavirus replicon particles. The first and second virus replicon particles are capable of expressing at least one target antigen. In a particular embodiment, the first and second virus replicon particles are capable of expressing the same target antigen or antigens. In another embodiment, the first and second virus replicon particles are capable of expressing at least one different target antigen.

In a particular aspect of the invention, a method of inducing or generating an immune response in a mammalian subject comprises (a) administering to the subject via a mucosal route of administration a first immunogenic composition comprising one or more virus replicon vectors or particles, the vectors or particles comprising at least one antigen; and (b) administering to the subject via a systemic route of administration a second immunogenic composition comprising one or more virus replicon vectors or particles, the vectors or particles comprising at least one antigen, thereby inducing or generating an immune response in the subject. In another aspect, the method of inducing or generating an immune response in a mammalian subject comprises (a) administering to the subject via a systemic route of administration a first immunogenic composition comprising one or more virus replicon vectors or particles, the vectors or particles comprising at least one antigen; and (b) administering to the subject via a mucosal route of administration a second immunogenic composition comprising one or more virus replicon vectors or particles, the vectors or particles comprising at least one antigen, thereby inducing an immune response in the subject. The virus replicon vectors and particles encode one or more (at least one) target antigen. The first and second immunogenic compositions may comprise the same or different virus replicon vectors or particles. In a particular embodiment, the first and second immunogenic compositions comprise virus replicon vectors or particles that are alphavirus replicon vectors or particles. In one embodiment, the virus replicon vectors and particles of the first and second immunogenic compositions are capable of expressing the same target antigen or antigens. In another embodiment, the virus replicon particles of the first and second immunogenic compositions are capable of expressing at least one different target antigen.

In certain embodiments, the virus replicon particles and immunogenic compositions described herein are administered to prime a mammalian subject. Priming, as used herein, means any method whereby a first immunization with the virus replicon particles or immunogenic compositions described herein permits the generation of an immune response to the target antigen or antigens upon a second immunization with virus replicon particles or immunogenic compositions described herein comprising at least one same antigen or antigens, wherein the second immune response is greater than that achieved where the first immunization is either not provided or where the first immunization administered contains a vector or particle which does not express the antigen or antigens. Priming encompasses regimens which include a single dose or multiple dosages, administered hourly, daily, weekly, monthly or yearly. In a particular embodiment, priming (or priming immunization) comprises at least two administrations (comprising one or more dose or dosage). For example, in a particular embodiment, priming by administration of one or more virus replicon particle or immunogenic composition described herein, via a mucosal route of administration, entails at least two (e.g., 2, 3, 4, 5, 6, 7 or more) mucosal administrations (comprising one or more dose or dosage) of the virus replicon particle(s) or immunogenic composition(s). Similarly, priming by administration of one or more virus replicon particle or immunogenic composition described herein, via a systemic route of administration, entails at least two (e.g., 2, 3, 4, 5, 6, 7 or more) systemic administrations (comprising one or more dose or dosage) of the virus replicon particle(s) or immunogenic composition(s). The time interval between mucosal and systemic administrations can be hours, days, weeks, months or years. Further, in certain embodiments, the repeated steps can be performed using the same or different virus replicon particles or immunogenic compositions.

In other embodiments, the virus replicon particles and immunogenic compositions described herein are administered as a booster to boost the immune response achieved after priming of the mammalian subject. Virus replicon particles or immunogenic compositions administered as a booster are administered some time after priming. Virus replicon particles or immunogenic compositions administered as a booster comprise at least one same antigen administered by the priming step. In a particular embodiment, boosting (or boosting immunization) is about two (2) to twenty-seven (27) weeks after priming (or priming immunization). Boosting encompasses regimens which include a single dose or multiple dosages, administered hourly, daily, weekly, monthly or yearly. In certain embodiments, boosting (or boosting immunization) comprises at least one administration. In other embodiments, boosting (or boosting immunization) comprises at least two administrations (comprising one or more dose or dosage). For example, in such instance, in a particular embodiment, boosting by administration of one or more virus replicon particle or immunogenic composition described herein, via a mucosal route of administration, entails at least two (e.g., 2, 3, 4, 5, 6, 7 or more) mucosal administrations (comprising one or more dose or dosage) of the virus replicon particle(s) or immunogenic composition(s). Similarly, in such instance, boosting by administration of one or more virus replicon particle or immunogenic composition described herein, via a systemic route of administration, entails at least two (e.g., 2, 3, 4, 5, 6, 7 or more) systemic administrations (comprising one or more dose or dosage) of the virus replicon particle(s) or immunogenic composition(s). The time interval between mucosal and systemic administrations can be hours, days, weeks, months or years. Further, in certain embodiments, the repeated steps can be performed using the same or different virus replicon particles or immunogenic compositions.

In other aspects of the invention, virus replicon particles can be used for a first series (comprising one or more dose or dosage) of mucosal and/or systemic immunizations (priming immunizations) followed by a second series (comprising one or more dose or dosage) of immunizations with DNA-based, bacterial or viral delivery systems or protein-based vaccines (boosting immunizations). In another aspect, DNA-based, bacterial or viral delivery systems or protein-based vaccines can be used for a first series (comprising one or more dose or dosage) of immunizations (priming immunizations) followed by a second series (comprising one or more dose or dosage) of immunizations with virus replicon particles (boosting immunizations). The priming or boosting immunizations with the replicon particles or DNA-based, bacterial or viral based or protein-based vaccines can be through a mucosal or a systemic or a simultaneous mucosal and systemic route of immunization. Thus, these mucosal/systemic prime-systemic/mucosal boost methods can be used to induce or generate an immune response to a wide variety of antigens.

Mucosal administration can be, for example, oral, intranasal, intragastric, pulmonary, intestinal, rectal, ocular and vaginal routes. Intranasal or oral administration is preferred. Systemic administration can be, for example, intramuscular. The mucosally and/or systemically administered compositions described herein can further comprise one or more additional agents such as adjuvants and/or delivery vehicles.

Antigens suitable for use in the invention can be derived from a pathogen, such as bacteria or a virus, or from a tumor. Bacterial antigens suitable for use in the invention include antigens derived from, for example, *Neisseria meningitidis,* subgroups A, B and or C, *Haemophilus influenzae, Streptococcus pneumoniae* and/or *Streptococcus agalactiae.* Viral antigens suitable for use in the invention include antigens derived from, for example, hepatitis A virus (HAV), human immunodeficiency virus (HIV), respiratory syncytial virus (RSV), parainfluenza virus (PIV), influenza, hepatitis B virus (HBV), herpes simplex virus (HSV), hepatitis C virus (HCV) and/or human papilloma virus (HPV).

In the methods described herein, the immune response can be a humoral and/or cellular immune response, a systemic immune response (e.g., IgG or cytokine production), a mucosal immune response (e.g., IgA or cytokine production) or a combination of systemic and mucosal responses.

In the methods described herein, the mucosally and systemically administered virus vectors and particles can comprise sequences encoding antigens from the same pathogen (e.g., bacteria, virus and/or tumor). In certain embodiments, the same virus vectors or particles are administered mucosally and systemically. In other embodiments, different alphavirus particles (e.g., by having different antigens from the same pathogen, different forms of the antigens, antigens from different pathogens and/or different alphavirus) are administered mucosally and systemically.

In the methods described herein, the immunogenic compositions may further comprise one or more polypeptide antigens and/or one or more polynucleotides encoding one or more antigens (e.g., alphavirus, poxvirus and/or adenovirus replicons and/or vectors). These polypeptide and/or polynucleotides may be administered separately (prior to or subsequent to) or concurrently with (in the same or different compositions) the alphavirus replicon particle-containing compositions.

In the methods described herein, one or more polypeptide antigens and/or one or more polynucleotides encoding one or more antigens (e.g., alphavirus replicon, poxvirus and/or adenovirus replicons and/or vectors) may be administered (mucosally or systemically) to the subject instead of or in addition to the compositions comprising alphavirus replicon particles.

The present invention also provides a kit for inducing or generating an immune response in a mammal. The kit comprises (i) a first composition which comprises a first viral replicon particle and which is formulated for mucosal administration to the mammal and (ii) a second composition which comprises a second virus replicon particle and which is formulated for systemic administration to the mammal, for sequential administration in either order. Virus replicon particles include alphavirus replicon particles, adenovirus replicon particles and poxvirus replicon particles. The first and second virus replicon particle can be the same or different viral replicon particles. The first and second compositions are capable of expressing at least one target antigen. In a particular embodiment, the first and second compositions are capable of expressing the same target antigen or antigens. In another embodiment, the first and second compositions are capable of expressing at least one different target antigen. The kit can comprise single or multiple doses of the first composition, of the second composition or of both first and second compositions. Thus, in a particular embodiment, to facilitate repeat administrations, the kit can comprise a plurality of vials for one or both compositions, each vial comprising the dose to be administered to the subject at each administration. The kit can further comprise instructions for use of the kit. In other embodiments, the kit can also comprise an applicator for administering the first composition to the mammal via a mucosal route and/or an applicator for administering the second composition to the mammal via a systemic route.

Thus, the invention includes, but is not limited to, the following numbered embodiments:

. 1. A method of generating an immune response in a subject, comprising
(a) mucosally administering a first immunogenic composition comprising one or more alphavirus replicon particles, the alphavirus replicon particles comprising at least one polynucleotide encoding an antigen; and
(b) systemically administering a second immunogenic composition comprising one or more alphavirus replicon particles, the alphavirus replicon particles comprising at least one polynucleotide encoding an antigen, thereby inducing an immune response in the subject.

2. The method of 1, wherein the mucosal administration is selected from the group consisting of intranasally, intrarectally and intravaginally.

3. The method of 1, wherein the systemic administration is intramuscular.

4. The method of any one of 1 to 3, wherein step (a) is performed at least two times.

5. The method of any one of 1 to 3, wherein step (a) is performed at least three times.

6. The method any one of 1 to 5, wherein step (b) is performed at least two times.

7. The method of any one of 1 to 6, wherein at least one alphavirus replicon particle is derived from Sindbis (SIN).

8. The method of any one of 1 to 6, wherein at least one alphavirus replicon particle is derived from Venezuelan equine encephalitis (VEE).

9. The method of any one of 1 to 6, wherein at least one alphavirus replicon particle is derived from Semliki Forest virus (SFV).

10. The method of any one of 1 to 6, wherein at least one alphavirus replicon particle is a chimeric VEE/SIN replicon particle.

11. The method of any one of 1 to 10, wherein at least one antigen is a viral antigen.

12. The method of 11, wherein the viral antigen is derived from HIV, SIV or FIV.

13. The method of 12, wherein the antigen is derived from a gag, env or pol polypeptide.

14. The method of 11, wherein the viral antigen is derived from a virus selected from the group consisting of an influenza virus, a respiratory syncytial virus (RSV), a parainfluenza virus (PIV) and a hepatitis C virus (HCV).

15. The method of any one of 1 to 10, wherein at least one antigen is a bacterial antigen.

16. The method of 15, wherein the bacterial antigen is derived from *Neisseria meningitidis.*

17. The method of 16, wherein the bacterial antigen is derived from the group consisting of *Neisseria meningitidis,* subgroup B and *Neisseria meningitidis,* subgroup C.

18. The method of 15, wherein the bacterial antigen is derived from a *Streptococcus spp.*

19. The method of any one of 1 to 10, wherein at least one antigen is a tumor antigen.

20. The method of any one of 1 to 19, wherein the first and second immunogenic compositions comprise the same antigen(s).

21. The method of any one of 1 to 29, wherein the first and second immunogenic compositions comprise different antigen(s).

22. The method of 21, wherein the different antigen(s) are derived from the same pathogen.

23. The method of claim 21, wherein the different antigen(s) are derived from different pathogens.

24. The method of any one of 1 to 23, wherein the first and/or second immunogenic compositions further comprise(s) an additional delivery vehicle.

25. The method of 24, wherein the delivery vehicle comprises a microparticle.

26. The method of any one of 1 to 25, wherein the first and/or second immunogenic compositions further comprise(s) an adjuvant.

27. The method of any one of 1 to 26, wherein the first and/or second immunogenic compositions further comprise(s) one or more polypeptide antigens.

28. The method of any one of 1 to 27, wherein the immune response is a systemic immune response.

29. The method of any one of 1 to 27, wherein the immune response is a mucosal immune response.

30. The method of any one of 1 to 27, wherein the immune response is a systemic and mucosal immune response.

31. The method of any one of 1 to 30, wherein step (a) precedes step (b).

32. The method of any one of 1 to 30, wherein step (b) precedes step (a).

33. The method of any one of 1 to 27, further comprising administering one or more polypeptide antigens or one or more polynucleotides encoding one or more antigens to the subject.

34. The method of 33, wherein the polynucleotides are carried on alphavirus replicon vectors, poxvirus replicons and/or vectors or adenovirus replicons and/or vectors.

These and other aspects of the present invention will become evident upon reference to the following detailed description and attached drawings. In addition, various references are set forth below which describe in more detail certain procedures or compositions (e.g., alphavirus replicon particles, etc.).

### BRIEF DESCRIPTION OF THE DRAWINGS

FIG. 1 is a graph depicting the percent of peripheral blood mononuclear cells (PMBC) infected with VEE-GFP replicon particles (left column); SIN-GFP replicon particles (middle column); or VEE/SIN-GFP replicon particles (right column).

FIG. 2 is a graph depicting infection of CD11b⁺, CD14⁺ and CD20-' cells in PBMC by VEE-GFP replicon particles (white bars); SIN-GFP replicon particles (black bars); or VEE/SIN-GFP replicon particles (gray bars).

FIG. 3 is a graph depicting expression of GFP in PBMC containing VEE-GFP replicon particles (left column); SIN-GFP replicon particles (middle column); or VEE/S1N-GFP replicon particles (right column).

FIG. 4 is a graph depicting gp140 IgG serum titers of the 4 animals of Group 1 of Study Design #2.

FIG. 5 is a graph depicting gap 140 IgG serum titers of the 4 animals of Group 2 of Study Design #2.

FIG. 6 is a graph depicting gp 140 IgG serum titers of the 4 animals of Group 3 of Study Design #2.

FIG. 7 is a graph depicting gp 140 IgG titers in vaginal washes of the 4 animals of Group 1 of Study Design #2.

FIG. 8 is a graph depicting gp140 IgG titers in vaginal washes of the 4 animals of Group 2 of Study Design #2.

FIG. 9 is a graph depicting gp140 IgG titers in vaginal washes of the 4 animals of Group 3 of Study Design #2.

FIG. 10 is a graph depicting gp 140 IgA serum titers of the 4 animals of Group 1 of Study Design #2.

FIG. 11 is a graph depicting gp140 IgA serum titers of the 4 animals of Group 2 of Study Design #2.

FIG. 12 is a graph depicting gp 140 IgA serum titers of the 4 animals of Group 3 of Study Design #2.

FIG. 13 is a graph depicting gp 140 IgA titers in vaginal washes of the 4 animals of Group 1 of Study Design #2.

FIG. 14 is a graph depicting gp140 IgA titers in vaginal washes of the 4 animals of Group 2 of Study Design #2.

FIG. 15 is a graph depicting gp140 IgA titers in vaginal washes of the 4 animals of Group 3 of Study Design #2.

FIG. 16 is a graph depicting gp 140 IgA titers in saliva of the 4 animals of Group 1 of Study Design #2.

FIG. 17 is a graph depicting gp140 IgA titers in saliva of the 4 animals of Group 2 of Study Design #2.

FIG. 18 is a graph depicting gp140 IgA titers in saliva of the 4 animals of Group 3 of Study Design #2.

### DETAILED DESCRIPTION OF THE INVENTION

The practice of the present invention will employ, unless otherwise indicated, conventional methods of chemistry, biochemistry, molecular biology, immunology and pharmacology, within the skill of the art. Such techniques are explained fully in the literature. See, e.g., Gennaro, A.R. (ed.), Remington's Pharmaceutical Sciences, 20th edition (Mack Publishing Company, 2000); Colowick, S. and Kaplan, N. (eds.), Methods In Enzymology (Academic Press, Inc., 1984); and Weir, D.M. (ed.), Weir's Handbook of Experimental Immunology, 5th edition, (Blackwell Publishers, 1996); Sambrook, J., et al., Molecular Cloning: A Laboratory Manual, 3rd edition (Cold Spring Harbor Laboratory Press, 2001); Birdi, K.S. (ed.), Handbook of surface and Colloidal Chemistry, 2nd edition (CRC Press, 2002); Ausubel, F.M. et al. (eds.), Short Protocols in Molecular Biology, 5th ed. (Current Protocols, 2002); Ream, W. and Field, K.G., Molecular Biology Techniques: An Intensive Laboratory Course (Academic Press, 1999); Newton, C.R. & Graham, A. (eds.), PCR (Introduction to Biotechniques Series), 2nd ed. (BIOS Scientific Publishers, 1997); Fields, B.N. et al. (eds.), Fields Virology, 4th edition (Lippincott Williams & Wilkins, 2001).

All publications, patents and patent applications cited herein, whether *supra* or *infra,* are incorporated herein by reference in their entireties.

As used in this specification and the appended claims, the singular forms "a," "an" and "the" include plural references unless the content clearly dictates otherwise. Thus, for example, reference to "an antigen" includes a mixture of two or more such agents.

Prior to setting forth the invention definitions of certain terms that will be used hereinafter are set forth.

A "polynucleotide" is a nucleic acid molecule that encodes a biologically active (e.g., immunogenic or therapeutic) protein or polypeptide. Depending on the nature of the polypeptide encoded by the polynucleotide, a polynucleotide can include as little as 10 nucleotides, e.g., where the polynucleotide encodes an antigen. Furthermore, a "polynucleotide" can include both double- and single-stranded sequences and refers to, but is not limited to, cDNA from viral, prokaryotic or eukaryotic MRNA, genomic RNA and DNA sequences from viral (e.g. RNA and DNA viruses and retroviruses) or prokaryotic DNA, and especially synthetic DNA sequences. The term also captures sequences that include any of the known base analogs of DNA and RNA, and includes modifications such as deletions, additions and substitutions (generally conservative in nature), to the native sequence, so long as the nucleic acid molecule encodes a therapeutic or antigenic protein. These modifications may be deliberate, as through site-directed mutagenesis, or may be accidental, such as through mutations of hosts that produce the antigens. Modifications of polynucleotides may have any number of effects including, for example, facilitating expression of the polypeptide product in a host cell.

The terms "polypeptide" and "protein" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include postexpression modifications of the polypeptide, for example, glycosylation, acetylation, phosphorylation and the like. Furthermore, for purposes of the present invention, a "polypeptide" refers to protein- that includes modifications, such as deletions, additions and substitutions (generally conservative in nature), to the native sequence, so long as the protein maintains the desired activity. These modifications may be deliberate, as through site-directed mutagenesis, or may be accidental, such as through mutations of hosts that produce the proteins or errors due to PCR amplification. Furthermore, modifications may be made that have one or more of the following effects: reducing toxicity; facilitating cell processing (e.g., secretion, antigen presentation, etc.); and facilitating presentation to B-cells and/or T-cells.

An "alphavirus replicon vector," "RNA replicon vector," "replicon vector" or "replicon" refers to a nucleic acid molecule that is capable of directing its own amplification or self replication *in vivo,* within a target cell. *See, e.g.,* U.S. Patent Nos. 6,767,669; 6,465,634; 6,458,560; 6,451,592; 6,426,196; 6,391,632; 6,376,236; 6,342,372; 6,329,201; 6,242,259; 6,105,694; 6,015,686; 5,843,723; 5,814,482; and 5,789,245. It will be apparent that, through the years, several terms including alphavirus vector, alphavirus vector construct, alphavirus replicon, alphavirus RNA replicon, alphavirus vector replicon, Eukaryotic Layered Vector Initiation System (ELVIS), alphavirus plasmid replicon and the like have emerged to describe alphavirus replicon vectors.

A "recombinant alphavirus particle" or "alphavirus replicon particle" refers to a virion-like structural unit containing an alphavirus RNA vector replicon. Generally, a recombinant alphavirus particle comprises one or more alphavirus structural proteins, a lipid envelope and an RNA vector replicon. Preferably, the recombinant alphavirus particle contains a nucleocapsid structure that is contained within a host cell-derived lipid bilayer, such as a plasma membrane, in which alphaviral-encoded envelope glycoproteins are embedded. The particle may also contain other components (e.g., targeting elements, other viral structural proteins, or other receptor binding ligands) that direct the tropism of the particle from which the alphavirus was derived. Alphavirus replicon particles can be made from one or more alphaviruses, including, but not limited to, Sindbis (SIN), Venezuelan equine encephalitis (VEE) and/or Semliki Forest virus (SFV). *See, e.g.,* U.S. Patent Nos. 6,770,283; 6,376,236; 6,015,694; 6,531,135; and 6,521,235. Chimeric alphavirus replicon particles (i.e., having sequences derived from more than one alphavirus) are described for examples in U.S. Patent Publication Nos. 2003/0232324 and 2003/0148262.

An "antigen" refers to a molecule containing one or more epitopes (either linear, conformational or both) that will stimulate a host's immune system to make an innate, humoral and/or cellular antigen-specific response. The term is used interchangeably with the term "immunogen." Normally, an epitope will include between about 3-15, generally about 5-15 amino acids. A B-cell epitope is normally about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes as well as tumor antigens, including extracellular domains of cell surface receptors and intracellular portions that may contain T-cell epitopes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide that expresses an antigen or antigenic determinant *in vivo,* such as in gene therapy and DNA immunization applications, is also included in the definition of antigen herein.

Epitopes of a given protein can be identified using any number of epitope mapping techniques, well known in the art. See, e.g., Morris, G.E. (ed.), Epitope Mapping Protocols (Methods in Molecular Biology), Vol. 66 (Humana Press, 1996). For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al. (1984) Proc. Natl. Acad. Sci. USA 81:3998-4002; Geysen et al. (1986) Mol. Immunol. 23:709-715.

Similarly, conformational epitopes are readily identified by determining spatial conformation of amino acids such as by, e.g., x-ray crystallography and nuclear magnetic resonance. See, e.g., *Epitope Mapping Protocols,* supra.

For purposes of the present invention, antigens can be derived from tumors and/or any of several known or yet uncharacterized viruses, bacteria, parasites and fungi, non-limiting examples of which are described more fully below. The term also intends any of the various tumor antigens or any other antigen to which an immune response is desired. Furthermore, for purposes of the present invention, an "antigen" refers to a protein that includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts that produce the antigens. Thus, antigens (and polynucleotides encoding these antigens) can be physically derived from a wild-type organism and/or produced recombinantly or synthetically, for example, based on known sequences.

The term "derived from" is used to identify the source of a molecule (e.g., polynucleotide, polypeptide, alphavirus replicon particle). A first polynucleotide is "derived from" second polynucleotide if it has the same or substantially the same base pair sequence as a region of the second polynucleotide, its cDNA, complements thereof, or if it displays sequence identity as described above. Thus, a viral sequence or polynucleotide is "derived from" a particular virus (e.g., species) if it has (i) the same or substantially the same sequence as the virus sequence or (ii) displays sequence identity to polynucleotides of that virus as described above.

A first polypeptide is "derived from" a second polypeptide if it is (i) encoded by a first polynucleotide derived from a second polynucleotide, or (ii) displays sequence identity to the second polypeptides as described above. Thus, a viral antigen is "derived from" a particular virus polypeptide if it is (i) encoded by an open reading frame of a polynucleotide of that virus (viral polynucleotide), or (ii) displays sequence identity, as described above, and/or antigenic functionality to polypeptide from which it was derived. Similarly, an alphavirus replicon particle is "derived from" one or more alphaviruses if it is (i) encoded by an open reading frame of a polynucleotide of that alphavirus, or (ii) displays sequence identity, as described above, to alphavirus(es) from which it was derived.

An "immunological response" or "immune response" to an antigen or composition is the development in a subject of an innate, humoral and/or a cellular immune response to an antigen present in the composition of interest. For purposes of the present invention, an "innate immune response" refers to induction of cytokines or chemokines from antigen presenting cells such as dendritic cells or epithelial cells or endothelial cells. An innate immune response may be generated by whole or sub- structures on the alphavirus replicon particles that bind to toll like receptors or other cellular receptors on cells involved in initiation of innate responses such as dendritic cells. A "humoral immune response" refers to an immune response mediated by antibody molecules, including secretory (IgA) or IgG molecules, while a "cellular immune response" is one mediated by T-lymphocytes and/or other white blood cells. One important aspect of cellular immunity involves an antigen-specific response by cytolytic T-lymphocytes ("GTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated:T-cells and/or other white blood cells, including those derived from CD4+ and CD8+T-cells. In addition, a chemokine response maybe induced by various white blood or endothelial cells in response to an administered antigen.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen to stimulate a cell-mediated immunological response may be determined by a number of assays, such as by lymphoproliferation (lymphocyte proliferation) assays, CTL cytotoxic cell assays, or by assaying for T-lymphocytes specific for the antigen in a sensitized subject. Such assays are well known in the art. See, e.g., Erickson et al. (1993) J. Immunol. 151:4189-4199; Doe et al. (1994) Eur. J. Immunol. 24:2369-2376. Recent methods of measuring cell-mediated immune response include measurement of intracellular cytokines or cytokine secretion by T-cell populations (e.g., by ELISPOT technique), or by measurement of epitope specific T-cells (e.g., by the tetramer technique) (reviewed by McMichael, A.J. and O'Callaghan, C.A. (1998) J. Exp. Med. 187(9):1367-1371; Mcheyzer-Williams, M.G. et al. (1996) Immunol. Rev. 150:5-21; Lalvani, A. et al (1997) J. Exp. Med. 186:859-865).

Thus, an immunological response as used herein may be one that stimulates CTLs, and/or the production or activation of helper T- cells. The production of chemokines and/or cytokines may also be stimulated. The antigen of interest may also elicit an antibody-mediated immune response. Hence, an immunological response may include one or more of the following effects: the production of antibodies (e.g., IgA or IgG) by B-cells; the activation of an innate immune responses by antigen presenting cells such as dendritic cells as well as epithelial cells or endothelial cells etc. comprising secretion of cytokines, chemokines or other factors; and/or the activation of suppressor, cytotoxic, or helper T-cells and/or (* T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

A "mucosal immune response" or "mucosal immunity" is meant the induction of a humoral (i.e., B cell) and/or cellular (i.e., T cell) response. Preferably, this immune response is specific for the antigen with which the mammalian subject was immunized. A humoral mucosal immune response may be assessed by measuring the antigen-specific antibodies present in the mucosal lavage in response to introduction of the desired antigen into the host. The antibody response, preferably, is composed primarily of IgA or IgG antibodies. A cellular mucosal immune response may be assessed by measuring the T cell response from lymphocytes isolated from the mucosal area (e.g., vagina or gastrointestinal tract) or from lymph nodes that drain from the mucosal area (for example genital area or gastrointestinal area).

An "immunogenic composition" is a composition that comprises an antigenic molecule where administration of the composition to a subject results in the development in the subject of a humoral and/or a cellular immune response to the antigenic molecule of interest. The immunogenic composition can be introduced directly into a recipient subject, such as by injection, inhalation, oral, intranasal or any other parenteral or mucosal (e.g., intra-rectally or intra-vaginally) route of administration.

By "subunit vaccine" is meant a vaccine composition that includes one or more selected antigens but not all antigens, derived from or homologous to, an antigen from a pathogen of interest such as from a virus, bacterium, parasite or fungus. Such a composition is substantially free of intact pathogen cells or pathogenic particles, or the lysate of such cells or particles. Thus, a "subunit vaccine" can be prepared from at least partially purified (preferably substantially purified) immunogenic polypeptides from the pathogen, or analogs thereof. The method of obtaining an antigen included in the subunit vaccine can thus include standard purification techniques, recombinant production, or synthetic production.

By "mucosal" or "via a mucosal route of administration" is meant introduction into the body via any mucosal surface, such as intranasally, orally, vaginally, rectally or the like. Mucosal administration is to be contrasted with "parenteral" or "systemic" administration, by which is meant administration to a non-mucosal surface, such as by subcutaneous, intramuscular, transcutaneous, intradermal, transdermal, intravenous or intraperitoneal administration.

By "co-administration" is meant introduction into a body or target cell of two or more compositions. The term includes administration in any order or concurrently.

An "immuno-modulatory factor" refers to a molecule, for example a protein that is capable of modulating (particularly enhancing) an immune response. Non-limiting examples of immunomodulatory factors include lymphokines (also known as cytokines), such as IL-6, TGF-β, IL-1, IL-2, IL-3, etc.); and chemokines (e.g., secreted proteins such as macrophage inhibiting factor). Certain cytokines, for example TRANCE, flt-3L, and a secreted form of CD40L are capable of enhancing the immunostimulatory capacity of APCs. Non-limiting examples of cytokines which may be used alone or in combination in the practice of the present invention include, interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-1α), interleukin-11 (IL-11), MIP-1γ, leukemia inhibitory factor (LIF), c-kit ligand, thrombopoietin (TPO), CD40 ligand (CD40L), tumor necrosis factor-related activation-induced cytokine (TRANCE) and flt3 ligand (flt-3L). Cytokines are commercially available from several vendors such as, for example, Genzyme (Framingham, MA), Amgen (Thousand Oaks, CA), R&D Systems and Immunex (Seattle, WA). The sequences of many of these molecules are also available, for example, from the GenBank database. It is intended, although not always explicitly stated, that molecules having similar biological activity as wild-type or purified cytokines (e.g., recombinantly produced or mutants thereof) and nucleic acid encoding these molecules are intended to be used within the spirit and scope of the invention. Immunomodulatory factors can be included with one, some or all of the compositions described herein or can be employed as separate formulations.

By the term "priming" is meant any method by which a first immunization using an antigen induces a higher level of immune response to the desired antigen upon subsequent re-immunization with the same antigen when compared with the immune response achieved where the first immunization is either not provided or where the first immunization administered contains a DNA vector which does not express the antigen. The term also includes multiple priming administrations. A priming administration can be administered systemically or mucosally. Preferably, the priming administration(s) is(are) by a mucosal route, for example intranasal (IN). The systemic administration includes any parenteral routes of administration characterized by physical breaching of a tissue of a subject and administration of the pharmaceutical composition through the breach in the tissue. In particular, parenteral administration is contemplated to include, but is not limited to, intradermal, transdermal, subcutaneous, intraperitoneal, intravenous, intraarterial, intramuscular, or intrasternal injection, intravenous, intraarterial, or kidney dialytic infusion techniques, and so-called "needleless" injections through tissue. Preferably, the systemic, parenteral administration is intramuscular injection. The route of administration of the vaccine may vary depending upon the identity of the pathogen or infection to be prevented or treated.

By "subject" is meant any member of the subphylum chordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats, and guinea pigs; birds, including domestic, wild and game birds such as chickens, turkeys and other gallinaceous birds, ducks, geese, and the like. The term does not denote a particular age. Thus, both adult and newborn individuals are intended to be covered. The system described above is intended for use in any of the above vertebrate species, since the immune systems of all of these vertebrates operate similarly.

By "vertebrate subject" is meant any member of the subphylum cordata, including, without limitation, mammals such as cattle, sheep, pigs, goats, horses, and humans; domestic animals such as dogs and cats; and birds, including domestic, wild and game birds such as cocks and hens including chickens, turkeys and other gallinaceous birds. The term does not denote a particular age. Thus, both adult and newborn animals are intended to be covered.

By "mammalian subject" is meant any male or female mammal. Preferably the mammalian subject is human. However, other primates as well as mammalian species, including without limitation, dogs, cats, cows, horses, pigs, sheep, goats, mice, rabbits and rats, etc. are also encompassed by this definition.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

The terms "effective amount" or "pharmaceutically effective amount" of a macromolecule and/or microparticle, as provided herein, refer to a nontoxic but sufficient amount of the macromolecule and/or microparticle to provide the desired response, such as an immunological response, and corresponding therapeutic effect, or in the case of delivery of a therapeutic protein, an amount sufficient to effect treatment of the subject, as defined below. As will be pointed out below, the exact amount required will vary from subject to subject, depending on the species, age, and general condition of the subject, the severity of the condition being treated, and the particular macromolecule of interest, mode of administration, and the like. An appropriate "effective" amount in any individual case may be determined by one of ordinary skill in the art using routine experimentation.

By "pharmaceutically acceptable" or "pharmacologically acceptable" is meant a material which is not biologically or otherwise undesirable, i.e., the material may be administered to an individual along with the microparticle formulation without causing any undesirable biological effects or interacting in a deleterious manner with any of the components of the composition in which it is contained.

By "physiological pH" or a "pH in the physiological range" is meant a pH in the range of approximately 7.2 to 8.0 inclusive, more typically in the range of approximately 7.2 to 7.6 inclusive.

As used herein, "treatment" refers to any of (i) the prevention of infection or reinfection, as in a traditional vaccine, (ii) the reduction or elimination of symptoms, and (iii) the substantial or complete elimination of the pathogen or disorder in question. Treatment may be effected prophylactically (prior to infection) or therapeutically (following infection).

### A. ALPHAVIRUS REPLICON PARTICLES

As noted above, any alphavirus replicon particle can be used in the methods described herein.

Generally, the recombinant alphavirus particle comprises one or more alphavirus structural proteins, a lipid envelope and an RNA vector replicon. In particular, the recombinant alphavirus particle generally contains a nucleocapsid structure that is contained within a host cell-derived lipid bilayer, such as a plasma membrane, in which one or more alphaviral envelope glycoproteins (e.g., E2, E1) are embedded.

### A.1. NUCLEOTIDE COMPONENTS

Furthermore, as noted above, particles as described herein typically include one or more polynucleotide sequences (e.g., RNA). When found in particles, these polynucleotides are surrounded by (and interact with) one or more structural proteins. Thus, the replicon particles described herein typically include a variety of nucleic acid sequences, both coding and non-coding sequences. Generally, the particles comprise less than a complete alphavirus genome (e.g., contain less than all of the coding and/or non-coding sequences contained in a genome of an alphavirus).

### A.1.A. NON-CODING SEQUENCES

Non-limiting examples of non-coding sequences include 5' sequences required for nonstructural protein-mediated amplification, a means for expressing a 3' proximal gene, subgenomic mRNA 5'-end nontranslated region (subgenomic 5' NTR), and 3' sequences required for nonstructural protein-mediated amplification (U.S. Patent Nos. 5,843,723; 6,015,694; and 5,814,482; International Publication Nos. WO 97/38087 and WO 00/61772).

Non-limiting examples of suitable 5' sequences include control elements such as native alphavirus 5'-end from homologous virus, native alphavirus 5'-end from heterologous virus, non-native DI alphavirus 5'-end from homologous virus, non-native DI alphavirus 5'-end from heterologous virus, non-alphavirus derived viral sequence (e.g., togavirus, plant virus), cellular RNA derived sequence (e.g., tRNA element) (e.g., Monroe et al. (1983) Proc. Natl. Acad. Sci. USA 80:3279-3283), mutations/deletions of any of the above sequences to reduce homology (See, e.g., Niesters et al. (1990) J. Virol. 64:4162-4168; Niesters et al. (1990) J. Virol. 64:1639-1647), and/or minimal 5' sequence in helpers (to approx. 200, 250, 300, 350,400 nucleotides).

The polynucleotide sequences of the replicon particles also generally include a means for expressing a 3' proximal gene (e.g., a heterologous sequence, polypeptide encoding sequence). Non-limiting examples of such means include control elements such as promoters and the like, for example, a native alphavirus subgenomic promoter from homologous virus, a native alphavirus subgenomic promoter from heterologous virus, a core alphavirus subgenomic promoter (homologous or heterologous), minimal sequences upstream or downstream from core subgenomic promoter, mutations/deletions/additions of core or native subgenomic promoter, a non-alphavirus derived compatible subgenomic promoter (e.g. plant virus), an internal ribosome entry site (IRES), and/or a ribosomal readthrough element (e.g., BiP).

Suitable subgenomic mRNA 5'-end nontranslated regions (subgenomic 5' NTR) include, but are not limited to, a native alphavirus subgenomic 5'NTR from homologous virus, a native alphavirus subgenomic 5'NTR from heterologous virus, a non-alphavirus derived viral 5'NTR (e.g., plant virus), a cellular gene derived 5'NTR (e.g., beta-globin), and/or sequences containing mutations, deletions, and/or additions to native alphavirus subgenomic 5'NTR.

Non-limiting examples of suitable 3' sequences required for nonstructural protein-mediated amplification include control elements such as a native alphavirus 3'-end from homologous virus, a native alphavirus 3'-end from heterologous virus, a non-native DI alphavirus 3'-end from homologous virus, a non-native DI alphavirus 3'-end from heterologous virus, a non-alphavirus derived viral sequence (e.g., togavirus, plant virus), a cellular RNA derived sequence, sequences containing mutations, deletions, or additions of above sequences to reduce homology (See, e.g., Kuhn et al. (1990) J. Virol. 64:1465-1476), minimal sequence in helpers to approx. (20, 30, 50, 100, 200 nucleotides) and/or sequences from cell-repaired 3' alphavirus CSE. A polyadenylation sequence can also be incorporated, for example, within 3'-end sequences. (See, e.g., George et al. (2000) J. Virol. 74:9776-9785).

### A.1.B. ALPHAVIRRUS CODING SEQUENCES

The particles described herein may also include one or more sequences coding for various alphavirus polypeptides, for example one or more of the non-structural (nsP1, nsP2, nsP3, nsP4) or structural (e.g., caspid, envelope) alphavirus polypeptides. *See, e.g.,* U.S. Patent Publication Nos. 2003/0232324 and 2003/0148262.

One or more of the nucleotide sequences of the replicon particles may be modified as compared to wild-type. Modifications to alphavirus coding sequences may include, but are not limited to nucleotide mutations, deletions, additions, or sequence substitutions, in whole or in part, such as for example using a hybrid nonstructural protein comprising sequences from one alphavirus and another virus (e.g., alphavirus, togavirus, plant virus). For example, in certain embodiments, there are one or more deletions in sequences encoding nonstructural protein gene(s). Such deletions may be in nonstructural protein (nsP) 1, 2, 3, or 4, as well as combinations of deletions from more than one nsP gene. For example, and not intended by way of limitation, a deletion may encompass at least the nucleotide sequences encoding VEE nsP1 amino acid residues 101-120, 450-470, 460-480, 470-490, or 480-500, numbered relative to the sequence in Kinney et al. (1989) Virology 170:19-30, as well as smaller regions included within any of the above.

In other embodiments, a deletion may encompass at least the sequences encoding VEE nsP2 amino acid residues 9-29,613-633, 650-670, or 740-760, as well as smaller regions included within any of the above. In another embodiment, a deletion may encompass at least the sequences encoding VEE nsP3 amino acid residues 340-370, 350-380, 360-390, 370-400, 380-410, 390-420, 400-430, 410-440, 420-450, 430-460, 440-470, 450-480, 460-490, 470-500, 480-510, 490-520, 500-530, or 488-522, as well as smaller regions included within any of the above. In another embodiment, the deletion may encompass at least the sequences encoding VEE nsP4 amino acid residues 8-28, or 552-570, as well as smaller regions included within any of the above. It should be noted that although the above amino acid ranges are illustrated using VEE as an example, similar types of deletions may be utilized in other alphaviruses. For example, in other embodiments, the modified non-structural proteins include a modification (e.g., deletion(s), addition(s) and/or substitution(s)) at a highly conserved location within an nsP4 of an alphavirus replicon.

By way of non-limiting example, the polymerase regions comprising nsP4 amino acids 368-400 of Sindbis virus (SIN), 375-407 of Semliki Forest virus (SFV), and 383-415 of Venezuelan equine encephalitis virus (VEE), as well as amino acids 462-494 of the 2a protein of the plant brome mosaic virus (BMV), have a high degree of sequence conservation and may serve as the target region for modification. Further, modifications to the adjacent amino acid sequence 1, 2 or 3 amino acids upstream or downstream from this region also are contemplated.

Generally, while amino acid numbering is somewhat different between alphaviruses, primarily due to slight differences in polyprotein lengths, alignments amongst or between sequences from different alphaviruses provides a means to identify similar regions in other alphaviruses (see representative alignment in Kinney et al..(1989) Virology 170:19-30). Preferably, the nonstructural protein gene deletions of the present invention are confined to a region or stretch of amino acids considered as non-conserved among multiple alphaviruses. In addition, conserved regions also may be subject to deletion.

The structural proteins surrounding (and in some cases, interacting with) the alphavirus replicon or vector polynucleotide component(s) can include both capsid and envelope proteins. In most instances, the polynucleotide component(s) are surrounded by the capsid protein(s), which form nucleocapsids. In turn, the nucleocapsid protein is surrounded by a lipid envelope containing the envelope protein(s). It should be understood although it is preferred to have both capsid and envelope proteins, both are not required.

Alphavirus capsid proteins and envelope proteins are described generally in Strauss et al. (1994) Microbiol. Rev. 58:491-562. The capsid protein is the N-terminal protein of the alphavirus structural polyprotein, and following processing from the polyprotein, interacts with alphavirus RNA and other capsid protein monomers to form nucleocapsid structures.

Alphavirus envelope glycoproteins (e.g., E2, E1) protrude from the enveloped particle as surface "spikes", which are functionally involved in receptor binding and entry into the target cell.

One or both of these structural proteins (or regions thereof) may include one or more modifications as compared to wild-type. "Hybrid" structural proteins (e.g., proteins containing sequences derived from two or more alphaviruses) also find use in the practice of the present invention. Hybrid proteins can include one or more regions derived from different alphaviruses. These regions can be contiguous or non-contiguous. Preferably, a particular region of the structural protein (e.g., a functional regions such as the cytoplasmic tail portion of the envelope protein or the RNA binding domain of the capsid protein) is derived from a first alphavirus. Any amount of the "remaining" sequences of the protein (e.g., any sequences outside the designated region) can be derived from one or more alphaviruses that are different than the first. It is preferred that between about 25% to 100% (or any percentage value therebetween) of the "remaining" portion be derived from a different alphavirus, more preferably between about 35% and 100% (or any percentage value therebetween), even more preferably between about 50% and 100% (or any percentage value therebetween). The sequences derived from the one or more different alphaviruses in the hybrid can be contiguous or non-contiguous, in other words, sequences derived from one alphavirus can be separated by sequences derived from one or more different alphaviruses.

The particle may also contain other components (e.g., targeting elements such as biotin, other viral structural proteins or portions thereof, hybrid envelopes, or other receptor binding ligands), which direct the tropism of the particle from which the alphavirus was derived. Generally, the interaction between alphavirus RNA and structural protein(s) necessary to efficiently form a replicon particle or nucleocapsid may be an RNA-protein interaction between a capsid protein and a packaging signal (or packaging sequence) contained within the RNA.

When used to generate an immune response, the alphavirus replicon particles will also contain a sequence encoding at least one antigen. Such antigens are discussed in detail below.

### A.1.c. PRODUCTION OF ALPHAVIRUS REPLICON PARTICLES

The chimeric alphavirus replicon particles according to the present invention may be produced using a variety of published methods. Such methods include, for example, transient packaging approaches, such as the co-transfection of in vitro transcribed replicon and defective helper RNA(s) (Liljestrom (1991) Bio/Technology 9:1356-1361; Bredenbeek et al. (1993) J. Viol 67:6439-6446; Frolov et al. (1997) J. Virol. 71:2819-2829; Pushko et al. (1997) Virology 239:389-401; U.S. Patent Nos. 5,789,245 and 5,842,723) or plasmid DNA-based replicon and defective helper constructs (Dubensky et al. (1996) J. Virol. 70:508-519), as well as introduction of alphavirus replicons into stable packaging cell lines (PCL) (Polo et al. (1999) Proc. Natl. Acad. Sci. USA 96:4598-4603; U.S. Patent Nos. 5,789,245; 5,842,723; and 6,015,694; International Publication Nos. WO 97138087; WO 99/18226; WO 00/61772; and WO 00/39318).

In preferred embodiments, stable alphavirus packaging cell lines are utilized for replicon particle production. The PCL may be transfected with in vitro transcribed replicon RNA, transfected with plasmid DNA-based replicon (e.g., ELVIS vector), or infected with a seed stock of replicon particles, and then incubated under conditions and for a time sufficient to produce high titer packaged replicon particles in the culture supernatant. In particularly preferred embodiments, PCL are utilized in a two-step process, wherein as a first step, a seed stock of replicon particles is produced by transfecting the PCL with a plasmid DNA-based replicon. A much larger stock of replicon particles is then produced in the second step, by infecting a fresh culture of the PCL with the seed stock. This infection may be performed using various multiplicities of infection (MOI), including a MOI=0.01, 0.05, 0.1, 0.5, 1.0, 3, 5, or 10. Preferably infection is performed at a low MOI (e.g., less than 1). Replicon particles at titers even >10⁸ infectious units (IU)/ml can be harvested over time from PCL infected with the seed stock. In addition, the replicon particles can subsequently be passaged in yet larger cultures of nave PCL by repeated low multiplicity infection, resulting in commercial scale preparations with the same high titer. Importantly, by using PCL of the "split" structural gene configuration, these replicon particle stocks may be produced free from detectable contaminating RCV.

Large-scale production of alphavirus replicon particles may be performed using a bioreactor. Preferably, the bioreactor is an external component bioreactor, which is an integrated modular bioreactor system for the mass culture, growth, and process control of substrate attached cells. The attachment and propagation of cells (e.g., alphavirus packaging cells) occurs in a vessel or chamber with tissue culture treated surfaces, and the cells are with fresh media for increased cell productivity. Monitoring and adjustments are performed for such parameters as gases, temperature, pH, glucose, etc., and crude vector is harvested using a perfusion pump. Typically, the individual components of an External Bioreactor separate external modules that are connected (i.e., via tubing). The external components can be pumps, reservoirs, oxygenators, culture modules, and other non-standard parts. A representative example of an External Component Bioreactor is the CellCube^{™} system (Coming, Inc).

In addition to using the external component bioreactor described herein, a more traditional Stir Tank Bioreactor may also be used, in certain instances, for alphavirus replicon particle production. In a Stir Tank Bioreactor, the alphavirus packaging cells may be unattached to any matrix (i.e., floating in suspension) or attached to a matrix (e.g., poly disks, micro- or macro carriers, beads). Alternatively, a Hollow Fiber Culture System may be used.

Following harvest, crude culture supernatants containing the alphavirus replicon particles may be clarified by passing the harvest through a filter (e.g., 0.2 pM, 0.45 uM, 0.65 11M, 0.8 p.M pore size). Optionally, the crude supernatants may be subjected to low speed centrifugation prior to filtration to remove large cell debris. Within one embodiment, an endonuclease (e.g., Benzonase, Sigma #E8263) is added to the preparation of alphavirus replicon particles before or after a chromatographic purification step to digest exogenous nucleic acid. Further, the preparation may be concentrated prior to purification using one of any widely known methods (e.g., tangential flow filtration).

Crude or clarified alphavirus replicon particles may be concentrated and purified by chromatographic techniques (e.g., ion exchange chromatography, size exclusion chromatography, hydrophobic interaction chromatography, affinity chromatography). Two or more such purification methods may be performed sequentially. In preferred embodiments, at least one step of ion exchange chromatography is performed and utilizes a ion exchange resin, such as a tentacle ion exchange resin, and at least one step of size exclusion chromatography is performed.

Briefly, clarified alphavirus replicon particle filtrates may be loaded onto a column containing a charged ion exchange matrix or resin (e.g., cation or anion exchange). The matrix or resin may consist of a variety of substances, including but not limited to cross-linked agarose, cross linked polystyrene, cross linked styrene, hydrophilic polyether resin, acrylic resin, and methacrylate based resin. The ion exchanger component may comprise, but is not limited to, a cationic exchanger selected from the list consisting of sulphopropyl cation exchanger, a carboxymethyl cation exchanger, a sulfonic acid exchanger, a methyl sulfonate cation exchanger, and an SO.sub.3-exchanger. In other embodiments, the ion exchanger component may comprise, but is not limited to, an anionic exchanger selected from the list consisting of DEAE, TMAE, and DMAE. Most preferably, ion exchange chromatography is performed using a tentacle cationic exchanger, wherein the ion exchange resin is a methacrylate-based resin with an SO₃-cation exchanger (e.g., FRACTOGEL^{™} EDM SO₃)-

The replicon particles may be bound to the ion exchange resin followed by one or more washes with buffer containing a salt (e.g., 250 mM or less NaC1). Replicon particles then may be eluted from the column in purified form using a buffer with increased salt concentration. In preferred embodiments, the salt concentration is a least 300 mM, 350 mM, 400 mM, 450 mM or 500 mM. Elution may be monitored preferably by a spectrophotometer at 280 nm, but also by replicon titer assay, transfer of expression (TOE) assay, or protein gel analysis with subsequent Coomassie staining or Western blotting.

The higher salt elution buffer subsequently may be exchanged for a more desirable buffer, for example, by dilution in the appropriate aqueous solution or by passing the particle-containing eluate over a molecular exclusion column. Additionally, the use of a molecular size exclusion column may also provide, in certain instances, further purification. For example, in one embodiment Sephacryl S-500 or S-400 (Pharmacia) chromatography may be used as both a buffer exchange as well as to further purify the fractions containing the replicon particles eluted from an ion exchange column. Using this particular resin, the replicon particles generally are eluted in the late void volume and show improvement in the level of purity as some of the contaminants are smaller in molecular weight and are retained on the column longer. However, alternative resins of different compositions as well as size exclusion could also be used that might yield similar or improved results. In these strategies, larger-sized resins such as Sephacryl S-1000 could be incorporated that would allow the replicon particles to enter into the matrix and thus be retained longer, allowing fractionation.

### B. ANTIGENS

The methods described herein can involve mucosal and systemic administration of one or more alphavirus replicon particles, each particle comprising one or more polynucleotides encoding an antigen derived from a bacterium, a virus, a prion, a tumor or other disease-causing angent.

For purposes of the present invention, any antigen can be used. Antigens can be derived from any of several known viruses, bacteria, parasites and fungi, as well as any of the various tumor antigens or any other antigen to which an immune response is desired. Furthermore, for purposes of the present invention, an "antigen" refers to a protein that includes modifications, such as deletions, additions and substitutions (generally conservative in nature), to the native sequence, so long as the protein maintains the ability to elicit an immunological response. These modifications may be deliberate, as through site-directed mutagenesis, or may be accidental, such as through mutations of hosts that produce the antigens.

Antigens for use in the practice of the present invention include polypeptide antigens derived from pathogens that infect or are transmitted through mucosal surfaces. Non-limiting representative examples of pathogens transmitted through mucosal surfaces and antigens derived therefrom include antigens derived from bacterial pathogens (*e.g., Neisseria meningitidis, Streptococcus agalactia, Haemophilus influenzae, Streptococcus pneumoniae,* chlamydia, gonorrhea and syphilis), viral pathogens (e.g., Human Immunodeficiency Virus ("HIV"), Hepatitis B and C Virus ("HBV" and "HCV", respectively), Human Papiloma Virus ("HPV"), Herpes Simplex Virus ("HSV"), and the like), as well as parasitic, fungal and cancer antigens. For a discussion of *Chlamydia pneumoniae* and *Chlamydia trachomatis,* see Kalman et al. (1999) Nature Genetics 21:385-389; Read et al. (2000) Nucleic Acids Research 28:1397-1406; Shirai et al. (2000) J. Infect. Dis. 181(Suppl.3):S524-S527; International Publication Nos. WO 99127105; WO 00/27994; WO 00/37494; and WO 99/28457.

As utilized within the context of the present invention, "immunogenic portion" refers to a portion of the respective antigen that is capable, under the appropriate conditions, of causing an immune response (i.e., cell-mediated or humoral). "Portions" may be of variable size, but are preferably at least 9 amino acids long, and may include the entire antigen. Cell-mediated immune responses may be mediated through Major Histocompatability Complex ("MHC") class I presentation, MHC Class II presentation, or both. As will be evident to one of ordinary skill in the art, various immunogenic portions of the antigens described herein may be combined in order to induce an immune response when administered as described herein.

Furthermore, the immunogenic portion(s) may be of varying length, although it is generally preferred that the portions be at least 9 amino acids long and may include the entire antigen. Immunogenicity of a particular sequence is often difficult to predict, although T cell epitopes may be predicted utilizing computer algorithms such as TSITES (MedImmune, Maryland), in order to scan coding regions for potential T-helper sites and CTL sites. From this analysis, peptides are synthesized and used as targets in an *in vitro* cytotoxic assay. Other assays, however, may also be utilized, including, for example, ELISA, which detects the presence of antibodies against the newly introduced vector, as well as assays which test for T helper cells, such as gamma-interferon assays, IL-2 production assays and proliferation assays.

Immunogenic portions of any antigen may also be selected by other methods. For example, the HLA A2.1 transgenic mouse has been shown to be useful as a model for human T-cell recognition of viral antigens. Briefly, in the influenza and hepatitis B viral systems, the murine T cell receptor repertoire recognizes the same antigenic determinants recognized by human T cells. In both systems, the CTL response generated in the HLA A2.1 transgenic mouse is directed toward virtually the same epitope as those recognized by human CTLs of the HLA A2.1 haplotype (Vitiello et al. (1991) J. Exp. Med. 173:1007-1015; Vitiello et al. (1992) Abstract of Molecular Biology of Hepatitis B Virus Symposia)*.*

Additional immunogenic portions may be obtained by truncating the coding sequence at various locations including, for example, to include one or more epitopes from the various regions, for example, of the HIV genome or one or more MenB epitopes. As noted above, such domains include structural domains such as *Gag, Gag-polymerase, Gag-protease, reverse transcriptase (RT), integrase (IN)* and *Env.* The structural domains are often further subdivided into polypeptides, for example, p55, p24, p6 *(Gag);* p160, p10, p15, p31, p65 (*pol, prot, RT and IN);* and gp160, gp120 and gp41 (*Env*)*.* Additional epitopes of HIV and other sexually transmitted diseases are known or can be readily determined using methods known in the art. Also included in the invention are molecular variants of such polypeptides, for example as described in International Publication Nos. WO 00/39302; WO 00/39304; and WO 00/39303.

Antigens for use in the practice of the invention include, but are not limited to, one or more of the antigens set forth below, or antigens derived from one or more of the pathogens set forth below. The antigen(s) may be used alone or in any combination of antigens (see, e.g., International Publication No. WO 02/00249 describing the use of combinations of bacterial antigens). The combinations may include multiple antigens from the same pathogen, multiple antigens from different pathogens or multiple antigens from the same and from different pathogens. Thus, bacterial,
viral, tumor and/or other antigens may be included in the same composition or may be administered to the same subject separately.

It is generally preferred that combinations of antigens used to raise an immune response be used in combinations. Immunization against multiple pathogens or antigens is advantageous, both for parenteral delivery (where the number of administrations is reduced) and for mucosal delivery because patient compliance is improved and transport/storage of medicines is facilitated. Immunization(s), as described herein, can be used prophylactically or therapeutically.

### B.1. BACTERIAL ANTIGENS

Bacterial antigens suitable for use in the invention include proteins, polysaccharides, lipopolysaccharides and outer membrane vesicles which may be isolated, purified or derived from a bacteria. In addition, bacterial antigens may include bacterial lysates and inactivated bacteria formulations. Bacteria antigens may be produced by recombinant expression. Bacterial antigens preferably include epitopes which are exposed on the surface of the bacteria during at least one stage of its life cycle. Bacterial antigens are preferably conserved across multiple serotypes. Bacterial antigens include antigens derived from one or more of the bacteria set forth below as well as the specific antigen examples identified below.

*Neisseria meningitides: Meningitides* antigens may include proteins (such as those identified in International Publication Nos. WO 99/24578; WO 99/36544; WO 99/57280; WO 00/22430; WO 96/29412; Tettelin et al. (2000) Science 287:1849-1815; and Pizza et al. (2000) Science 287:1816-1820), saccharides (including a polysaccharide, oligosaccharide or lipopolysaccharide), or outer-membrane vesicles (International Publication No. WO O1/52885; Bjune et al. (1991) Lancet 338(8775):1093-1096; Fuskasawa et al. (1999) Vaccine 17:2951-2958; and Rosenqist et al. (1998) Dev. Biol. Strand 92:323-333) purified or derived from *N. m*e*ningitides* serogroup such as A, C, W135, Y, and/or B. Meningitides protein antigens may be selected from adhesions, autotransporters, toxins, Fe acquisition proteins, and membrane associated proteins (preferably integral outer membrane protein).

*Streptococcus pneumoniae: Streptococcus pneumoniae* antigens may include a saccharide (including a polysaccharide or an oligosaccharide) and/or protein from *Streptococcus pneumoniae.* Saccharide antigens may be selected from serotypes 1, 2, 3, 4, 5, 6B, 7F, 8, 9N, 9V, 10A, 11A, 12F, 14, 15B, 17F, 18C, 19A, 19F, 20, 22F, 23F, and 33F. Protein antigens maybe selected from a protein identified in International Publication No. WO 98l18931; International Publication No. WO 98/18930; U.S. Patent No. 6,699,703; U.S. Patent No. 6,800,744; International Publication No. WO 97/43303; and International Publication No. WO 97/37026. *Streptococcus pneumoniae* proteins may be selected from the Poly Histidine Triad family (PhtX), the Choline Binding Protein family (CbpX), CbpX truncates, LytX family, LytX truncates, CbpX truncate-LytX truncate chimeric proteins, pneumolysin (Ply), PspA, PsaA, Sp128, Sp101, Sp130, Sp125 or Sp133. See also Watson et al. (2000) Pediatr. Infect. Dis. J. 19:331-332; Rubin et al. (2000) Pediatr. Clin. North Am. 47:269-284; and Jedrzejas et al. (2001) Microbiol. Mol. Biol. Rev. 65:187-207.

*Streptococcus pyogenes (Group A Streptococcus):* Group A Streptococcus antigens may include a protein identified in International Publication No. WO 02/34771 or International Publication No. WO 2005/032582 (including GAS 40), fusions of fragments of GAS M proteins (including those described in International Publication No. WO 02/094851; Dale (1999) Vaccine 17:193-200; and Dale (1996) Vaccine 14(10): 944-948), fibronectin binding protein (Síbl), Streptococcal heme-associated protein (Shp), and Streptolysin S (SagA). See also Dale et al. (1999) Infect. Dis. Clin. North Arn. 13:227-243; and Ferretti et al. (2001) Proc. Natl. Acad. Sci. USA 98:4658-4663.

*Moraxella catarrhalis:* Moraxella antigens include antigens identified in International Publication Nos. WO 02/18595 and WO 99/58562, outer membrane protein antigens (HMW-OMP), C-antigen, and/or LPS. See also McMichael (2000) Vaccine 19 Suppl. 1:S101-S107.

*Bordetella pertussis:* Pertussis antigens include petussis holotoxin (PT) and filamentous haemagglutinin (FHA) from *B. pertussis,* optionally also combination with pertactin and/or agglutinogens 2 and 3 antigen. See, e.g., Gusttafsson et al. (1996) N. Engl. J. Med. 334:349-355; and Rappuoli et al. (1991) TIBTECH 9:232-238*.*

*Staphylococcus aureus:* Staph aureus antigens include *S. aureus* type 5 and 8 capsular polysaccharides optionally conjugated to nontoxic recombinant *Pseudomonas aeruginosa* exotoxin A, such as StaphVAX™, or antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin). See e.g. Kuroda et al. (2001) Lancet 357:1225-1240.

*Staphylococcus epidermis: S. epidermidis* antigens include slime-associated antigen (SAA).

*Clostridium tetani* (Tetanus): Tetanus antigens include tetanus toxoid (TT), preferably used as a carrier protein in conjunction/conjugated with the compositions of the present invention.

*Cornynebacterium diphtheriae* (Diphtheria): Diphtheria antigens include diphtheria toxin, preferably detoxified, such as CRM₁₉₇. Additionally antigens capable of modulating, inhibiting or associated with ADP ribosylation are contemplated for combination/co-administration/conjugation with the compositions of the present invention. The diphtheria toxoids may be used as carrier proteins.

*Haemophilus influenzae B (Hib):* Hib antigens include a Hib saccharide antigen. See, e.g., Costantino et al. (1999) Vaccine 17:1251-1263).

*Pseudomonas aeruginosa:* Pseudomonas antigens include endotoxin A, Wzz protein, *P. aeruginosa* LPS, more particularly LPS isolated from PAO1 (O5 serotype), and/or Outer Membrane Proteins, including Outer Membrane Proteins F (OprF) (Price et al. (2001) Infect. Immun. 69(5):3510-3515).

*Legionella pneumophila.* Bacterial antigens may be derived from Legionella pneumophila.

*Streptococcus agalactiae (Group B Streptococcus):* Group B Streptococcus antigens include a protein or saccharide antigen identified in International Publication No. WO 02/34771, WO 03/093306, WO 04/041157 or WO 2005/002619 (including proteins GBS 80, GBS 104, GBS 276 and GBS 322, and including saccharide antigens derived from serotypes Ia, Ib, Ia/c, II, III, IV, V, VI, VII and VIII). See also Schuchat (1999) Lancet 353:51-56; and GB Patent Application Nos. 0026333.5; 0028727.6; and 015640.7.

*Neiserria gonorrhoeae:* Gonorrhoeae antigens include Por (or porin) protein, such as PorB (*see* Zhu et al. (2004) Vaccine 22:660-669), a transferring binding protein, such as TbpA and TbpB (see Price et al. (2004) Infect. Immun. 71(1):277-283), a opacity protein (such as Opa), a reduction-modifiable protein (Rmp), and outer membrane vesicle (OMV) preparations (*see* Plante et al. (2000) J. Infect. Dis. 182:848-855); see also e.g. International Publication Nos. WO99/24578, WO99/36544, WO99/57280 and WO02/079243).

*Chlamydia trachomatis:* Chlamydia trachomatis antigens include antigens derived from serotypes A, B, Ba and C (agents of trachoma, a cause of blindness), serotypes L₁, L₂ & L₃ (associated with Lymphogranuloma venereum), and serotypes, D-K. Chlamydia trachomas antigens may also include an antigen identified in International Publication No. WO 00/37494, WO 03/049762, WO 03/068811 or WO 05/002619, including PepA (CT045), LcrE (CT089), ArtJ (CT381), DnaK (CT396), CT398, OmpH-like (CT242), L7/L12 (CT316), OmcA (CT444), AtosS (CT467), CT547, Eno (CT587), HrtA (CT823), and MurG (CT761).

*Treponema pallidum* (Syphilis): Syphilis antigens include TmpA antigen.

*Haemophilus ducreyi* (causing chancroid): Ducreyi antigens include outer membrane protein (DsrA).

*Enterococcus faecalis or Enterococcus faecium:* Antigens include a trisaccharide repeat or other *Enterococcus* derived antigens provided in U.S. Patent No. 6,756,361.

*Helicobactef-pylori:* H pylori antigens include Cag, Vac, Nap, HopX, HopY and/or urease antigen. See, e.g., International Publication Nos. WO 93/18150; WO 99/53310; and WO 98/04702.

*Staphylococcus saprophyticus:* Antigens include the 160 kDa hemagglutinin of *S. saprophyticus* antigen.

*Yersinia enterocolitica* Antigens include LPS (Infect Immun. 2002 August; 70(8): 4414).

*E. coli:* E. coli antigens may be derived from enterotoxigenic *E. coli* (ETEC), enteroaggregative *E. coli* (EAggEC), diffusely adhering *E. coli* (DAEC), enteropathogenic *E. coli* (EPEC), and/or enterohemorrhagic *E. coli* (EHEC). °

*Bacillus anthracis* (anthrax): *B. anthracis* antigens are optionally detoxified and may be selected from A-components (lethal factor (LF) and edema factor (EF)), both of which can share a common B-component known as protective antigen (PA).

*Yersinia pestis* (plague): Plague antigens include F1 capsular antigen (Grosfeld et al. (2003) Infect. Immun. 71(l):374-383), LPS (Fields et al. (1999) Infect. Immun. 67(10):5395-5408), *Yersinia pestis* V antigen (Hill et al. (1997) Infect. Immun. 65(11):4476-4482).

*Mycobacterium tuberculosis:* Tuberculosis antigens include lipoproteins, LPS, BCG antigens, a fusion protein of antigen 85B (Ag85B) and/or ESAT-6 optionally formulated in cationic lipid vesicles (Olsen et al. (2004) Infect. Immun. 72(10):6148-6150), Mycobacterium tuberculosis (Mtb) isocitrate dehydrogenase associated antigens (Banerjee et al. (2004) Proc. Natl. Acad. Sci. USA 101(34):12652-12657) and/or MPT51 antigens (Suzuki et al. (2004) Infect. Immun. 72(7):3829-3837).

*Rickettsia:* Antigens include outer membrane proteins, including the outer membrane protein A and/or B (OmpB) (Chao et al. (2004) Biochim. Biophys. Acta. 1702(2):145-152), LPS and surface protein antigen (SPA) (Carl et al. (1989) J. Autoimmun. 2 Suppl:81-91).

*Listeria monocytogenes:* Bacterial antigens may be derived from Listeria monocytogenes.

*Chlamydia pneumoniae:* Antigens include those identified in International Publication No. WO 02/02606.

*Vibrio cholerae:* Antigens include proteinase antigens, LPS, particularly lipopolysaccharides of *V. cholerae* II, O1 Inaba 0-specific polysaccharides, *V. cholera* O139, antigens of IEM108 vaccine (Liang et al. (2003) Infect. Immun. 71(10):5498-5504) and/or Zonula occludens toxin (Zot).

*Salmonella typhi* (typhoid fever): Antigens include capsular polysaccharides preferably conjugates (Vi, i.e. vax-TyVi).

*Borrelia burgdorferi* (Lyme disease): Antigens include lipoproteins (such as OspA, OspB, Osp C and Osp D), other surface proteins such as OspE-related proteins (Erps), decorin-binding proteins (such as DbpA), and antigenically variable VI proteins. , such as antigens associated with P39 and P13 (an integral membrane protein (Noppa et al. (2003) Infect. Immun. 69(5):3323-3334), :V1sE Antigenic Variation Protein (Lawrenz et al. (1999) J. Clin. Microbiol. 37(12)3997-4004).

*Porphyromonas gingivalis:* Antigens include *P. gingivalis* outer membrane protein (OMP). See, e.g., Ross et al. (2001) Vaccine 19:4135-4132.

*Klebsiella:* Antigens include an OMP, including OMP A, or a polysaccharide optionally conjugated to tetanus toxoid.

Further bacterial antigens may be capsular antigens, polysaccharide antigens or protein antigens of any of the above. Further bacterial antigens may also include an outer membrane vesicle (OMV) preparation. Additionally, antigens include live, attenuated, and/or purified versions of any of the aforementioned bacteria. Antigens may be derived from gram-negative or gram-positive bacteria. Antigens may be derived from aerobic or anaerobic bacteria.

Additionally, any of the above bacterial-derived saccharides (polysaccharides, LPS, LOS or oligosaccharides) can be conjugated to another agent or antigen, such as a carrier protein (for example CRM₁₉₇). Such conjugation may be direct conjugation effected by reductive amination of carbonyl moieties on the saccharide to amino groups on the protein, as provided in U.S. Patent No. 5,360,897 and Roy et al. (1984) Can. J. Biochem. Cell. BioL 62(5):270-275. Alternatively, the saccharides can be conjugated through a linker, such as, with succinamide or other linkages provided in Bioconjugate Techniques (1996) and CRC, Chemistry of Protein Conjugation and Cross-Linking (1993).

### B.2. VIRAL ANTIGENS

Viral antigens for use in the invention include inactivated (or killed) virus, attenuated virus, split virus formulations, purified subunit formulations, viral proteins which may be isolated, purified or derived from a virus, and Virus Like Particles (VLPs). Viral antigens may be derived from viruses propagated on cell culture or other substrate. Alternatively, viral antigens may be expressed recombinantly. Viral antigens preferably include epitopes which are exposed on the surface of the virus during at least one stage of its life cycle. Viral antigens are preferably conserved across multiple serotypes or isolates. Viral antigens include antigens derived from one or more of the viruses set forth below as well as the specific antigens examples identified below.

*Orthomyxovirus:* Viral antigens may be derived from an Orthomyxovirus, such as Influenza A, B and C. Orthomyxovirus antigens may be selected from one or more of the viral proteins, including hemagglutinin (HA), neuraminidase (NA), nucleoprotein (NP), matrix protein (M1), membrane protein (M2), one or more of the transcriptase components (PB1, PB2 and PA). Preferred antigens include HA and NA. Numerous HA subtypes of influenza A have been identified (Kawaoka et al. (1990) Virol. 179:759-767; Webster et al., Antigenic variation among type A influenza viruses," pp. 127-168. In P. Palese and D.W. Kingsbury (ed.), Genetics of influenza viruses (NY: Springer-Verlag).

Influenza antigens may be derived from interpandemic (annual) flu strains. Alternatively influenza antigens may be derived from strains with the potential to cause pandemic a pandemic outbreak (i.e., influenza strains with new haemagglutinin compared to the haemagglutinin in currently circulating strains; or influenza strains which are pathogenic in avian subjects and have the potential to be transmitted horizontally in the human population, or influenza strains which are pathogenic to humans).

*Paramyxoviridae* viruses: Viral antigens may be derived from Paramyxoviridae viruses, such as Pneumoviruses (RSV), Paramyxoviruses (PIV) and Morbilliviruses (Measles).

*Pneumovirus:* Viral antigens may be derived from a Pneumovirus, such as Respiratory syncytial virus (RSV), Bovine respiratory syncytial virus, Pneumonia virus of mice, and Turkey rhinotracheitis virus. Preferably, the Pneumovirus is RSV. Pneumovirus antigens may be selected from one or more of the following proteins, including surface proteins Fusion (F), Glycoprotein (G) and Small Hydrophobic protein (SH), matrix proteins M and M2, nucleocapsid proteins N, P and L and nonstructural proteins NS1 and NS2. Preferred Pneumovirus antigens include F, G and M. See e.g., Johnstone et al. (2004) J. Gen. Virol. 85(Pt 11):3229-3238). Pneumovirus antigens may also be formulated in or derived from chimeric viruses. For example, chimeric RSV/PIV viruses may comprise components of both RSV and PIV.

*Paramyxovirus:* Viral antigens may be derived from a Paramyxovirus, such as Parainfluenza virus types 1- 4 (P1V), Mumps, Sendai viruses, Simian virus 5, Bovine parainfluenza virus and Newcastle disease virus. Preferably, the Paramyxovirus is PIV or Mumps. Paramyxovirus antigens may be selected from one or more of the following proteins: Hemagglutinin -Neuraminidase (HN), Fusion proteins F1 and F2, Nucleoprotein (NP), Phosphoprotein (P), Large protein (L), and Matrix protein (M). Preferred Paramyxovirus proteins include HN, F1 and F2. Paramyxovirus antigens may also be formulated in or derived from chimeric viruses. For example, chimeric RSV/PIV viruses may comprise components of both RSV and PIV. Commercially available mumps vaccines include live attenuated mumps virus, in either a monovalent form or in combination with measles and rubella vaccines (MMR).

*Morbillivirus:* Viral antigens may be derived from a Morbillivirus, such as Measles. Morbillivirus antigens may be selected from one or more of the following proteins: hemagglutinin (H), Glycoprotein (G), Fusion factor (F), Large protein (L), Nucleoprotein (NP), Polymerase phosphoprotein (P), and Matrix (M). Commercially available measles vaccines include live attenuated measles virus, typically in combination with mumps and rubella (MMR).

*Picornavirus:* Viral antigens may be derived from Picomaviruses, such as Enteroviruses, Rhinoviruses, Heparnavirus, Cardioviruses and Aphthoviruses. Antigens derived from Enteroviruses, such as Poliovirus, are preferred. Picomaviruses (e.g., polioviruses, etc.) are described, for example, in Sutter et al. (2000) Pediatr. Clin. North Am. 47:287-308; and 2inunerman and Spann (1999) Am. Farm. Physician 59:113-118, 125-126).

*Enterovirus:* Viral antigens may be derived from an Enterovirus, such as Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71. Preferably, the Enterovirus is poliovirus. Enterovirus antigens are preferably selected from one or more of the following Capsid proteins VP 1, VP2, VP3 and VP4. Commercially available polio vaccines include Inactivated Polio Vaccine (IPV) and Oral poliovirus vaccine (OPV)

*Heparnavirus:* Viral antigens may be derived from an Hepadnavirus, such as Hepatitis A virus (HAV). See, e.g., Bell et al. (2000) Pediatr. Infect. Dis. J. 19:1187-1188; and Iwarson (1995) APMIS 103:321-326. Commercially available HAV vaccines include inactivated HAV vaccine.

*Togavirus*: Viral antigens may be derived from a Togavirus, such as a Rubivirus, an Alphavirus, or an Arterivirus. Antigens derived from Rubivirus, such as Rubella virus, are preferred. Togavirus antigens may be selected from E1, E2, E3, C, NSP-1, NSPO-2, NSP-3 or NSP-4. Togavirus antigens are preferably selected from E1, E2 or E3. Commercially available Rubella vaccines include a live cold-adapted virus, typically in combination with mumps and measles vaccines (MMR).

*Flavivirus:* Viral antigens may be derived from a Flavivirus, such as Tick-borne encephalitis (TBE), Dengue (types 1, 2, 3 or 4), Yellow Fever, Japanese encephalitis, West Nile encephalitis, St. Louis encephalitis, Russian spring-summer encephalitis, Powassan encephalitis. Flavivirus antigens may be selected from PrM, M, C, E, NS-1, NS-2a, NS2b, NS3, NS4a, NS4b, and NS5. Flavivirus antigens are preferably selected from PrM, M and E. Commercially available TBE vaccine include inactivated virus

*Pestivirus:* Viral antigens may be derived from a Pestivirus, such as Bovine viral diarrhea (BVDV), Classical swine fever (CSFV) or Border disease (BDV).

*Hepadnavirus:* Viral antigens may be derived from a Hepadnavirus, such as Hepatitis B virus. See, e.g., Gerlich et al. (1990) Vaccine 8 Supp1:S63-68 & 79-80. Hepadnavirus antigens may be selected from surface antigens (L, M and S), core antigens (HBc, HBe). Additionally, Hepadnavirus antigens may be selected from the presurface sequences, pre-S 1 and pre-52. (formerly called pre-S), as well as combinations of the above, such as sAg/pre-S 1, sAg/pre-S2, sAg/pre-Sl/pre-S2, and pre-SI/pre-S2. See, *e.g*., "HBV Vaccines-from the laboratory to license: a case study" in Mackett, M. and Williamson, J.D., Human Vaccines and Vaccination, pp. 159-176, for a discussion of HBV structure; and U.S. Patent Nos. 4,722,840; 5,098,704; and 5,324,513; Beames et al. (1995) J. Virol. 69:6833-6838, Bimbaum et al. (1990) J. Virol. 64:3319-3330; and Zhou et al. (1991) J. Virol. 65:5457-5464*.* Commercially available HBV vaccines include subunit vaccines comprising the surface antigen S protein.

*Hepatitis C virus:* Viral antigens may be derived from a Hepatitis C virus (HCV). HCV antigens may be selected from one or more of E1 , E2, El/E2, NS345 polyprotein, NS 345-core polyprotein, core, and/or peptides from the nonstructural regions (Houghton et al., Hepatology (1991) 14:381).

*Rhabdovirus:* Viral antigens may be derived from a Rhabdovirus, such as a Lyssavirus (Rabies virus) and Vesiculovirus (VSV). Rhabdovirus (e.g., rabies virus, etc) are described, for example, in Dressen et al. (1997) Vaccine 15 Suppl:s2-6; MMWR Morb. Mortal Wkly Rep. 1998 Jan 16:47(1):12, 19). Rhabdovirus antigens may be selected from glycoprotein (G), nucleoprotein (N), large protein (L), nonstructural proteins (NS). Commercially available Rabies virus vaccine comprise killed virus grown on human diploid cells or fetal rhesus lung cells.

*Caliciviridae;* Viral antigens may be derived from Calciviridae, such as Norwalk virus, and Norwalk-like Viruses, such as Hawaii Virus and Snow Mountain Virus.

*Coronavirus:* Viral antigens may be derived from a Coronavirus, SARS, Human respiratory coronavirus, Avian infectious bronchitis (IBV) Mouse hepatitis virus (MHV), and Porcine transmissible gastroenteritis virus (TGEV). Coronavirus antigens may be selected from spike (S), envelope (E), matrix (M), nucleocapsid (N), and Hemagglutinin-esterase glycoprotein (HE). Preferably, the Coronavirus antigen is derived from a SARS virus. SARS viral antigens are described in International Publication No. WO 04/92360.

*Reovirus:* Viral antigens may be derived from a Reovirus, such as an Orthoreovirus, a Rotavirus, an Orbivirus, or a Coltivirus. Reovirus antigens may be selected from structural proteins λ1, λ2, λ3, µ1, µ2, σ1, σ2, or σ3, or nonstructural proteins σNS, µNS, or σ1s. Preferred Reovirus antigens may be derived from a Rotavirus. Rotavirus antigens may be selected from VP1, VP2, VP3, VP4 (or the cleaved product VP5 and VP8), NSP1, VP6, NSP3, NSP2, VP7, NSP4, or NSPS. Preferred Rotavirus antigens include VP4 (or the cleaved product VP5 and VP8), and VP7.

*Parvovirus:* Viral antigens may be derived from a Parvovirus, such as Parvovirus B 19. Parvovirus antigens may be selected from VP-1, VP-2, VP-3, NS-1 and NS-2. Preferably, the Parvovirus antigen is capsid protein VP-2.

*Delta hepatitis virus (HDV)*: Viral antigens may be derived HDV, particularly δ-antigen from HDV (see, e.g., U.S. Patent No. 5,378,814).

*Hepatitis E virus (HEV)*: Viral antigens may be derived from HEV.

*Hepatitis G virus (HGV):* Viral antigens may be derived from HGV.

*Human Herpesvirus:* Viral antigens may be derived from a Human Herpesvirus, such as Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8). Human Herpesvirus antigens may be selected from immediate early proteins (a), early proteins (β), and late proteins (γ). HSV antigens may be derived from HSV-1 or HSV-2 strains. HSV antigens may be selected from glycoproteins gB, gC, gD and gH, fusion protein (gB), or immune escape proteins (gC, gE, or gI). VZV antigens may be selected from core, nucleocapsid, tegument, or envelope proteins. A live attenuated VZV vaccine is commercially available. EBV antigens may be selected from early antigen (EA) proteins, viral capsid antigen (VCA), and glycoproteins of the membrane antigen (MA). CMV antigens may be selected from capsid proteins, envelope glycoproteins (such as gB and gH), and tegument proteins. (See, e.g. Chee et al., Cytomegaloviruses (J.K. McDougall, ed., Springer-Verlag, 1990) pp. 125-169, for a review of the protein coding content of cytomegalovirus; McGeoch et al. (1988) J. Gen. Virol. 69:1531-1574, for a discussion of the various HSV-1 encoded proteins; U.S. Patent No. 5,171,568 for a discussion of HSV-1 and HSV-2 gB and gD proteins and the genes encoding therefor; Baer et al. (1984) Nature 310:207-211, for the identification of protein coding sequences in an EBV genome; and Davison and Scott (1986) J. Gen. Virol. 67:1759-1816, for a review of VZV.)

*Papovaviruses:* Antigens may be derived from Papovaviruses, such as Papillomaviruses and Polyomaviruses. Papillomaviruses include HPV serotypes 1, 2, 4, 5, 6, 8, 11, 13, 16, 18, 31, 33, 35, 39, 41, 42, 47, 51, 57, 58, 63 and 65. Preferably, HPV antigens are derived from serotypes 6, 11, 16 or 18. HPV antigens may be selected from capsid proteins (L1) and (L2), or E1 - E7, or fusions thereof. HPV antigens are preferably formulated into virus-like particles (VLPs). Polyomyavirus viruses include BK virus and JK virus. Polyomavirus antigens may be selected from VP1, VP2 or VP3.

Retrovirus: Viral antigens may be derived from a Retrovirus, such as an Oncovirus, a Lentivirus or a Spumavirus. Oncovirus antigens may be derived, for example, from HTLV-1, HTLV-2, HTLV-5 or HTLV-11. Lentivirus antigens may be derived from HIV-1 (also known as HTLV-III, LAV, ARV, HTI,R, etc.) orHIV-2. Retrovirus antigens may be selected from gag, pol, env, tax, tat, rex, rev, nef, vif, vpu and vpr. HIV antigens may be selected from gag (e.g., p24gag and p55gag), env (e.g., gp160 and gp41), pol, tat, nef, rev, vpu, miniproteins (preferably p55 gag and gp140v delete). HIV antigens may be derived from one or more HIV strains, such as, for example, HIV_{IIIb}, HIV_{SF2}, HIV_{LAV}, HIV_{LAI}, HIV_{MN}, HIV-1_{CM235}, HIV-1_{US4}.

The gp 120 envelope proteins from any of the above HIV isolates, including members of the various genetic subtypes of HIV, are known and reported (see, e.g., Myers et al. (1992) Los Alamos Database, Los Alamos National Laboratory, Los Alamos, NM, Myers et al. (1990) Human Retroviruses and AIDS (Los Alamos, NM: Los Alamos National Laboratory); and Modrow et al. (1987) J. Virol. 61:570-578, for a comparison of the envelope sequences of a variety of HIV isolates) and antigens derived from any of these isolates will find use in the present methods. Other immunogenic proteins derived from any of the various HIV isolates, including any of the various envelope proteins such as gp160 and gp41, gag antigens such as p24gag and p55gag, as well as proteins derived from the pol region, are also suitable for use in the invention.

In addition, due to the large immunological variability that is found in different geographic regions for the open reading frame of HIV, particular combinations of antigens may be preferred for administration in particular geographic regions. Briefly, at least eight different subtypes of HIV have been identified and, of these, subtype B viruses are more prevalent in North America, Latin America and the Caribbean, Europe, Japan and Australia. Almost every subtype is present in sub-Saharan Africa, with subtypes A and D predominating in central and eastern Africa, and subtype C in southern Africa. Subtype C is also prevalent in India and it has been recently identified in southern Brazil. Subtype E was initially identified in Thailand, and is also present in the Central African Republic. Subtype F was initially described in Brazil and in Romania. The most recent subtypes described are G, found in Russia and Gabon, and subtype H, found in Zaire and in Cameroon. Group 0 viruses have been identified in Cameroon and also in Gabon. Thus, as will be evident to one of ordinary skill in the art, it is generally preferred to construct a vector for administration that is appropriate to the particular HIV subtype that is prevalent in the geographical region of administration. Subtypes of a particular region may be determined by two-dimensional double immunodiffusion or, by sequencing the HIV genome (or fragments thereof) isolated from individuals within that region.

As described above, also presented by HIV are various *Gag* and *Env* antigens. HIV-1 I *Gag* proteins are involved in many stages of the life cycle of the virus including, assembly, virion maturation after particle release, and early post-entry steps in virus replication. (Freed, E.O. (1998) Virol. 251:1-15).

*Env* coding sequences of the present invention include, but are not limited to, polynucleotide sequences encoding the following HIV-encoded polypeptides: gp160, gp140, and gp120 (see, e.g., U.S. Patent No. 5,792,459 for a description of the HIV-I_{SF2} ("SF2") Env polypeptide). The envelope protein of HIV-1 is a glycoprotein of about 160 kD (gp 160). During virus infection of the host cell, gp160 is cleaved by host cell proteases to form gp120 and the integral membrane protein, gp4l. The gp41 portion is anchored in (and spans) the membrane bilayer of virion, while the gp120 segment protrudes into the surrounding environment. As there is no covalent attachment between gp120 and gp41, free gp120 is released from the surface of virions and infected cells. Thus, gp160 includes the coding sequences for gp120 and gp41. The polypeptide gp41 is comprised of several domains including an oligomerization domain (OD) and a transmembrane spanning domain (TM). In the native envelope, the oligomerization domain is required for the non-covalent association of three gp41 polypeptides to form a trimeric structure: through non-covalent interactions with the gp41 trimer (and itself), the gp 120 polypeptides are also organized in a trimeric structure. A cleavage site (or cleavage sites) exists approximately between the polypeptide sequences for gp 120 and the polypeptide sequences corresponding to gp41. This cleavage site(s) can be mutated to prevent cleavage at the site. The resulting gp 140 polypeptide corresponds to a truncated form of gp160 where the transmembrane spanning domain of gp41 has been deleted. This gp140 polypeptide can exist in both monomeric and oligomeric (i.e. trimeric) forms by virtue of the presence of the oligomerization domain in the gp41 moiety and oligomeric form may be designed "o," for example "ogp140" refers to oligomeric gp140. In the situation where the cleavage site has been mutated to prevent cleavage and the transmembrane portion of gp41 has been deleted the resulting polypeptide product can be designated "mutated" gp140. As will be apparent to those in the field, the cleavage site can be mutated in a variety of ways. (See, also, International Publication No. WO 00/39302).

In certain embodiments, one or more of the antigens are derived from HIV. The genes of HIV are located in the central region of the proviral DNA and encode at least nine proteins divided into three major classes: (1) the major structural proteins, Gag, Pol and Env; (2) the regulatory proteins, Tat and Rev and (3) the accessory proteins, Vpu, Vpr, Vif and Nef. Although exemplified herein with relation to antigens obtained from HIV_{SF2,} sequence obtained from other HIV variants may be manipulated in similar fashion following the teachings of the present specification. Such other variants include, but are not limited to, Gag protein encoding sequences obtained from the isolates HIV_{IIIb}, HIV_{SF2}, HIV-I_{SF162}, HIV-1_{SF170,} HIV_{LAV}, HIV_{LAI}, HIV_{MN}, HIV-1_{CM235}, HIV-1_{US4}, other HIV-1 strains from diverse subtypes (e.g., subtypes, A through G, and 0), HIV-2 strains and diverse subtypes (e.g., HIV-2_{UC1} and HIV-2_{UC2}), and simian immunodeficiency virus (SIV). (See, e.g., Virology, 3rd Edition (W.K. Joklik (ed.) 1988); Fundamental Virology, 2nd Edition (B.N. Fields and D.M. Knipe (eds.) 1991); Virology, 3rd Edition (Fields, B.N., D.M. Knipe, P.M. Howley (eds.) 1996, Lippincott-Raven, Philadelphia, PA; for a description of these and other related viruses).

Further provided are antigens and microbes included in Vaccines, 4th Edition (Plotkin and Orenstein ed. 2004); Medical Microbiology 4th Edition (Murray et al. ed. 2002); Virology, 3rd Edition (W.K. Joklik ed. 1988); and Fundamental Virology, 2nd Edition (B.N. Fields and D.M. Knipe, eds. 1991).

### B.3. FUNGAL ANTIGENS

Fungal antigens for use in the invention may be derived from one or more of the fungi set forth below.

Fungal antigens may be derived from Dermatophytres, including: Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum var. album, var. discoides, var. ochraceum, Trichophyton violaceum and/or Trichophyton faviforme.

Fungal pathogens may be derived from Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium mameffei, Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp and Cladosporium spp.

### B.4. STD ANTIGENS

One or more antigens suitable for use in the invention may be derived from a sexually transmitted disease (STD). Such antigens may provide for prophylactis or therapy for one or more STD, such as chlamydia, genital herpes, hepatits (such as HCV), genital warts, gonorrhoea, syphilis and/or chancroid (See, WO00/15255). Antigens may be derived from one or more viral or bacterial STD's. Viral STD antigens for use in the invention may be derived from, for example, HIV, herpes simplex virus (HSV-1 and HSV-2), human papillomavirus (HPV), and hepatitis (HCV). Bacterial STD antigens for use in the invention may be derived from, for example, *Neiserria gonorrhoeae, Chlamydia trachomatis, Treponema pallidₗₗm, Haemophilus ducreyi, E. coli,* and *Streptococcus agalactiae.* Examples of specific antigens derived from these pathogens are described above.

### B.5. RESPIRATORY ANTIGENS

One or more antigens suitable for use in the invention may be derived from a pathogen which causes respiratory disease. For example, respiratory antigens may be derived from a respiratory virus such as Orthomyxoviruses (influenza), Pneumovirus (RSV), Paramyxovirus (PIV), Morbillivirus (measles), Togavirus (Rubella), VZV, and Coronavirus (SARS). Respiratory antigens may be derived from a bacteria which causes respiratory disease, such as *Streptococcus pneumoniae, Pseudomonas aeruginosa, Bordetella pertussis, Mycobacterium tuberculosis, Mycoplasma pneumoniae, Chlamydia pneumoniae, Bacillus anthracis,* and *Moraxella catarrhalis.* Examples of specific antigens derived from these pathogens are described above.

### B.6. PEDIATRIC ANTIGENS

One or more antigens suitable for use in the invention may include one or more antigens suitable for use in pediatric subjects. Pediatric subjects are typically less than about 3 years old, or less than about 2 years old, or less than about 1 years old. Pediatric antigens may be administered multiple times over the course of 6 months, 1, 2 or 3 years. Pediatric antigens may be derived from a virus which may target pediatric populations and/or a virus from which pediatric populations are susceptible to infection. Pediatric viral antigens include antigens, derived from one or more of Orthomyxovirus (influenza), Pneumovirus (RSV), Paramyxovirus (PIV and Mumps), Morbillivirus (measles), Togavirus (Rubella), Enterovirus (polio), HBV, Coronavirus (SARS), and Varicella-zoster virus (VZV), Epstein Barr virus (EBV). Pediatric bacterial antigens include antigens derived from one or more of *Streptococcus pneumoniae, Neisseria meningitides, Streptococcus pyogenes* (Group A Streptococcus), *Moraxella catarrhalis, Bordetella pertussis, Staphylococcus aureus, Clostridium tetani* (Tetanus), *Cornynebacterium diplatheriae* (Diphtheria), *Haemophilus influenzae B* (Hib), *Pseudomonas aeruginosa, Streptococcus agalactiae* (Group B Streptococcus), and *E*. *coli.* Examples of specific antigens derived from these pathogens are described above.

### B.7. ANTIGENS SUITABLE FOR USE IN ELDERLY OR IMMUNOCOMPROMISED

One or more antigens suitable for use in the invention may include one or more antigens suitable for use in elderly or immunocompromised individuals. Such individuals may need to be vaccinated more frequently, with higher doses or with adjuvanted formulations to improve their immune response to the targeted antigens. Antigens which may be targeted for use in Elderly or Immunocompromised individuals include antigens derived from one or more of the following pathogens: Neisseria meningitides, Streptococcus pneumoniae, Streptococcus pyogenes (Group A Streptococcus), Moraxella catarrhalis, Bordetella pertussis, Staphylococcus aureus, Staphylococcus epidermis, Clostridium tetani (Tetanus), Cornynebacterium diphtheriae (Diphtheria), Haemophilus influenzae B (Hib), Pseudomonas aeruginosa, Legionella pneumophila, Streptococcus agalactiae (Group B Streptococcus), Enterococcus faecalis, Helicobacter pylori, Clamydia pneumoniae, Orthomyxovirus (influenza), Pneumovirus (RSV), Paramyxovirus (PIV and Mumps), Morbillivirus (measles), Togavirus (Rubella), Enterovirus (polio), HBV, Coronavirus (SARS), Varicella-zoster virus (VZV), Epstein Barr virus (EBV), Cytomegalovirus (CMV). Examples of specific antigens derived from these pathogens are described above.

### B.8. ANTIGENS SUITABLE FOR USE IN ADOLESCENT VACCINES

One or more antigens suitable for use in the invention may include one or more antigens suitable for use in adolescent subjects. Adolescents may be in need of a boost of a previously administered pediatric antigen. Pediatric antigens which may be suitable for use in adolescents are described above. In addition, adolescents may be targeted to receive antigens derived from an STD pathogen in order to ensure protective or therapeutic immunity before the beginning of sexual activity. STD antigens which may be suitable for use in adolescents are described above.

### B.8. TUMOR ANTIGENS

One or more antigens suitable for use in the invention may include one or more tumor or cancer antigens. Tumor antigens can be, for example, peptide-containing tumor antigens, such as a polypeptide tumor antigen or glycoprotein tumor antigens. A tumor antigen can also be, for example, a saccharide-containing tumor antigen, such as a glycolipid tumor antigen or a ganglioside tumor antigen. The tumor antigen can further be, for example, a polynucleotide-containing tumor antigen that expresses a polypeptide-containing tumor antigen, for instance, an RNA vector construct or a DNA vector construct, such as plasmid DNA.

Tumor antigens for use in practice of the invention encompass a wide variety of molecules, such as (a) polypeptide-containing tumor antigens, including polypeptides (which can range, for example, from 8-20 amino acids in length, although lengths outside this range are also common), lipopolypeptides and glycoproteins, (b) saccharide-containing tumor antigens, including poly-saccharides, mucins, gangliosides, glycolipids and glycoproteins, and (c) polynucleotides that express antigenic polypeptides.

Tumor antigens can be, for example: (a) full length molecules associated with cancer cells, (b) homologs and modified forms of the same, including molecules with deleted, added and/or substituted portions, and (c) fragments of the same. Tumor antigens can be provided in recombinant form. Tumor antigens include, for example, class I-restricted antigens recognized by CD8+ lymphocytes or class II-restricted antigens recognized by CD4+ lymphocytes.

Numerous tumor antigens are known in the art, including, but not limited to: (a) cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors); (b) mutated antigens, for example, p53 (associated with various solid tumors, e.g., colorectal, lung, head and neck cancer), p21/Ras (associated with, e.g., melanoma, pancreatic cancer and colorectal cancer), CDK4 (associated with, e.g., melanoma), MUM1 (associated with, e.g., melanoma), caspase-8 (associated with, e.g., head and neck cancer), CIA 0205 (associated with, e.g., bladder cancer), HLA-A2-R1701, beta catenin (associated with, e.g., melanoma), TCR (associated with, e.g., T-cell non-Hodgkins lymphoma), BCR-abl (associated with, e.g., chronic myelogenous leukemia), triosephosphate isomerase, KIA 0205, CDC-27, and LDLR-FUT; (c) over-expressed antigens, for example, Galectin 4 (associated with, e.g., colorectal cancer), Galectin 9 (associated with, e.g., Hodgkin's disease), proteinase 3 (associated with, e.g., chronic myelogenous leukemia), WT 1 (associated with, e.g., various leukemias), carbonic anhydrase (associated with, e.g., renal cancer), aldolase A (associated with, e.g., lung cancer), PRAME (associated with, e.g., melanoma), HER-2/neu (associated with, e.g., breast, colon, lung and ovarian cancer), alpha-fetoprotein (associated with, e.g., hepatoma), KSA (associated with, e.g., colorectal cancer), gastrin (associated with, e.g., pancreatic and gastric cancer), telomerase catalytic protein, MUC-1 (associated with, e.g., breast and ovarian cancer), G-250 (associated with, e.g., renal cell carcinoma), p53 (associated with, e.g., breast, colon cancer), and carcinoembryonic antigen (associated with, e.g., breast cancer, lung cancer, and cancers of the gastrointestinal tract such as colorectal cancer); (d) shared antigens, for example, melanoma-melanocyte differentiation antigens such as MART-1/Melan A, gp100, MC1R, melanocyte-stimulating hormone receptor, tyrosinase, tyrosinase related protein-1/TRP1 and tyrosinase related protein-2/TRP2 (associated with, e.g., melanoma); (e) prostate associated antigens such as PAP, PSA, PSMA, PSH-P1, PSM-P1, PSM-P2, associated with e.g., prostate cancer; (f) immunoglobulin idiotypes (associated with myeloma and B cell lymphomas, for example); and (g) other tumor antigens, such as polypeptide- and saccharide-containing antigens including (i) glycoproteins such as sialyl Tn and sialyl Le^{x} (associated with, e.g., breast and colorectal cancer) as well as various mucins; glycoproteins may be coupled to a carrier protein (e.g., MUC-1 may be coupled to KLH), (ii) lipopolypeptides (e.g., MUC-1 linked to a lipid moiety), (iii) polysaccharides (e.g., Globo H synthetic hexasaccharide), which may be coupled to a carrier proteins (e.g., to KLH), and (iv) gangliosides such as GM2, GM12, GD2, GD3 (associated with, e.g., brain, lung cancer, melanoma), which also may be coupled to carrier proteins (e.g., KLH). Additional tumor antigens which are known in the art include p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KPl, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, and the like. These as well as other cellular components are described for example in U.S. Patent Publication No. 2002/0007173 and references cited therein.

Polynucleotide-containing antigens in accordance with the present invention typically comprise polynucleotides that encode polypeptide cancer antigens such as those listed above. Preferred polynucleotide-containing antigens include DNA or RNA vector constructs, such as plasmid vectors (e.g., pCMV), which are capable of expressing polypeptide cancer antigens in *vivo.*

Tumor antigens may be derived, for example, from mutated or altered cellular components. After alteration, the cellular components no longer perform their regulatory functions, and hence the cell may experience uncontrolled growth. Representative examples of altered cellular components include ras, p53, Rb, altered protein encoded by the Wilms' tumor gene, ubiquitin, mucin, protein encoded by the DCC, APC, and MCC genes, as well as receptors or receptor-like structures such as neu, thyroid hormone receptor, platelet derived growth factor (PDGF) receptor,
insulin receptor, epidermal growth factor (EGF) receptor, and the colony stimulating factor (CSF) receptor. These as well as other cellular components are described for example in U.S. Patent No. 5,693,522 and references cited therein.

Additionally, one or more bacterial and viral antigens may be used in conjunction with one or more tumor antigens for the treatment of cancer. In particular, carrier proteins, such as CRM₁₉₇, tetanus toxoid, or *Salmonella typhimurium* antigen can be used in conjunction/conjugation with compounds of the present invention for treatment of cancer. The cancer antigen combination therapies will show increased efficacy and bioavailability as compared with existing therapies.

Additional information on cancer or tumor antigens can be found in, for example, Moingeon (2001) Vaccine 19:1305-1326; Rosenberg (2001) Nature 411:380-384*;* Dermine et al. (2002) Brit. Med. Bull. 62:149-162; Espinoza-Delgado (2002) The Oncologist 7(suppl3):20-33; Davis .. et al. (2003) J. Leukocyte Biol. 23:3-29; Van den Eynde et al. (1995) Curr. Opin. Immunol 7:674-81; Rosenberg (1997) Immunol. Today 18:175-182; Offringa et al. (2000) Curr Opin. Immunol. 2:576-582; Rosenberg (1999) Immunity 10:281-287; Sahin et al. (1997) Curr. Opin. Immunol. 9:709-716; Old et al. (1998) J. Exp. Med. 187:1163-1167; Chaux et al. (1999) J. Exp. Med. 189:767-778; Gold et al. (1965) J. Exp. Med. 122:467-468; Livingston et al. (1997).Cancer Immunol. Immunother. 45:1-6; Livingston et al. (1997) Cancer Immunol. Immunother. 45:10-19; Taylor-Papadimitriou (1997) Immunol. Today 18:105-107; Zhao X-J et al. (1995) J. Exp. Med. 182:67-74; Theobald et al. (1995) Proc. Natl. Acad. Sci. USA 92:11993-11997; Gaudernack (1996) Immunotechnology 2:3-9; International Publication No. WO 91/02062; U.S. Patent No. 6,015,567; International Publication No. WO 01/08636; International Publication No. WO 96/30514; U.S. Patent No. 5,846,538; and U.S. Patent No. 5,869,445.

### C. DELIVERY

The methods described herein involve mucosal and systemic (parenteral) administrations, including, for example intravenous, intramuscular, intraperitoneal, subcutaneous, transcutaneous for systemic administration and oral, rectal, intraocular, aural or intranasal for mucosal administration.

Methods of systemic administration of compositions are well known and include, for example, (1) direct injection into the blood stream (e.g., intravenous administration); (2) direct injection into a specific tissue or tumor; (3) subcutaneous administration; (4) transcutaneous epidermal administration; (5) intradermal administration; (6) intraperitoneal administration; (7) transcutaneous administration (*e.g*., administering the vaccine on the skin surface which may or may not have been treated to remove a first layer of epithelial cells) and/or (8) intramuscular administration. Other modes of parenteral administration include pulmonary administration, suppositories, needle-less injection, transcutaneous and transdermal applications.

Similarly, methods of mucosal delivery are known in the art, for example as described in *Remington's, supra* and includes nasal, rectal, oral and vaginal delivery. For instance, tablets or capsules optionally enteric-coated, liquid, transgenic plants may be used for oral administration. Where the composition is for intranasal administration, it may be in the form of a nasal spray, nasal drops, gel or powder.

Other physical methods that may be useful for mucosal and/or systemic administration of compositions (e.g., alphavirus replicon particles, poxvirus particles, adenovirus particles, polypeptides, alphavirus replicon vectors, poxvirus vectors, adenovirus vectors, etc.) include, but are not limited to, lipofection (Felgner et al. (1989) Proc. Natl. Acad. Sci. USA 84:7413-7417), direct DNA injection (Acsadi et al. (1991) Nature 352:815-818); microprojectile bombardment (Williams et al. (1991) Proc. Natl. Acad. Sci. USA 88:2726-2730); liposomes of several types (*see, e.g.,* Wang et al. (1987) Proc. Natl. Acad. Sci. USA 84:7851-7855); CaPO₄ (Dubensky et al. (1984) Proc. Natl. Acad. Sci. USA 81:7529-7533); DNA ligand (Wu et al (1989) J. Biol. Chem. 264:16985-16987); administration of replicon particles.:alone; administration of nucleic acids alone (International Publication No. WO 90/11092); or administration of DNA linked to killed adenovirus (Curiel et al. (1992), Hum. Gene Ther. 3:147-154); via polycation compounds such as polylysine, utilizing receptor specific ligands; as well as with psoralen inactivated viruses such as Sendai or Adenovirus. Transcutaneous administration may include the use of a penetration enhancer, a barrier disruption agent or combinations thereof. See, *e.g*,. WO 99/43350. In addition, the administration may either be administered directly (*i.e.*, *in vivo*), or to cells that have been removed (*ex vivo*), and subsequently returned.

Dosage treatment may be a single dose schedule or a multiple dose schedule. The compositions can be administered in any order, for example a single mucosal administration followed by a single systemic administration; multiple mucosal administrations followed by a single systemic administration; multiple mucosal administrations followed by multiple system administrations; a single systemic administration followed by a single mucosal administration; multiple systemic administrations followed by a single mucosal administration; multiple systemic administrations followed by multiple mucosal administrations; mucosal (one or more) administration followed by systemic (one or more) administration followed by additional mucosal (one or more) administration(s); systemic (one or more) administration followed by mucosal (one or more) administration followed by additional systemic (one or more) administrations; concurrent administration; and the like.

As noted above, the methods described herein preferably involve at least one priming step followed by at least one boosting step. The priming and boosting steps involve administering one or more antigens to a mammalian subject. In preferred embodiments, the priming step(s) involves mucosal administration of at least one antigen-containing composition and the boosting step(s) involves systemic administration of at least one antigen-containing composition.

Mucosal delivery can be accomplished by aerosol, nebulizer, or by depositing a liquid in the nasal cavity. Alternatively, boosting may be by suppository, enema, or vaginal douche or other inhalation methods where direct immunization at a different mucosal surface of interest is desired. Similarly, systemic delivery can be accomplished by administration to any site which is not covered by mucosa (e.g., systemic administration excludes administration to intranasal, oral, vaginal, intratracheal, intestinal or rectal mucosal surfaces). In certain embodiments, the systemic administration is by parenteral routes of administration characterized by physical breaching of a tissue of a subject and administration of the pharmaceutical composition through the breach in the tissue. In particular, parenteral administration is contemplated to include, but is not limited to, intradermal, transdermal, subcutaneous, intraperitoneal, intravenous, intraarterial, intramuscular, or intrasternal injection, intravenous, intraarterial, or kidney dialytic infusion techniques, and so-called "needleless" injections through tissue. Preferably, the systemic, parenteral administration is intramuscular injection.

The priming and/or boosting compositions may be polypeptide and/or DNA vaccine compositions. Preferably, the priming and/or boosting vaccines comprise alphavirus replicon particles carrying nucleic acid encoding the antigen(s) of interest. Non-limiting examples of suitable alphavirus replicon particles include SIN, VEE and chimeric VEE/SIN replicon particles. In the Examples below, exemplary priming DNA vaccines are alphavirus replicon particles containing an HIV gene as the antigen, e.g. Env (gp120, gp140, g160), Gag, Prot, Pol, tat, rev, nef, vpr, vpu, vif or combinations thereof. In other embodiments, the priming and/or boosting compositions comprise poxvirus replicon particles. Non-limiting examples of poxvirus replicon particles include orthopoxviruses, parapoxviruses, avipoxviruses, caripoxviruses, leporipoxviruses, suipoxviruses, molluscipoxviruses and yatapoxviruses replicon particles. In yet other embodiments, the priming and/or boosting compositions comprise adenovirus replicon particles.

Optionally, the priming and/or boosting steps also include administering with the DNA vaccine composition, a suitable amount of any biologically active factor, such as cytokine, an interleukin, a chemokine, a ligands, and optimally combinations thereof, which, when administered with the DNA vaccine encoding an antigen, enhances the antigen-specific immune response compared with the immune response generated upon administration of the DNA vaccine encoding the antigen only.

Exemplary prime-boost methods are described in examples below in which a subject is mucosally primed and systemically boosted with an alphavirus replicon particle. According to this invention, the induction of an immune response (e.g., IgA and/or IgG) upon systemic (e.g., IM) boosting with an alphavirus replicon particle and systemic (IM) boosting can be significantly augmented by priming via mucosal immunization.

The interval between administrations will vary according to factors such as the age of the patient and the nature of the composition and these factors can be assessed by a physician. Administration of the first priming and boosting doses is generally separated by at least 2 weeks, typically at least 4 weeks. The methods of the invention may comprise more than one mucosal priming dose and/or more than one boosting dose; e.g., two or more priming doses followed by two or more booster doses. The term "memory" boost refers to any boosting dose given after the initial boost. The time at which the "memory" boost is administered can vary from hours (*e.g*., 1 to 72 hours or any timepoint therebetween) or days (*e.g*, 1 to 90 days or any timepoint therebetween) to months (*e.g*., 1 to 36 months or any timepoint therebetween) or even years after the initial boost. More than one memory boost may be administered at the same or varying time intervals with respect to each other. Identical or different immunogenic compositions may be used for each priming dose. Priming and boosting doses may be therefore distinguished by the route of administration, rather than by their timing.

In certain embodiments, the timing between administrations may be determined by evaluating the immune response and administering subsequent immunogenic compositions when the desired immune response is generated. Techniques of measuring immune responses in a subject are known and include but are not limited to, measurement of humoral or cellular immune responses in vaginal and/or nasal washes, fluids (lung, vaginal, nasal, etc.), or tissues (lung, etc.).

The mammal to whom the compositions are administered is typically primate, such as a human. The human may be a child or an adult. Suitable lower mammals may include mice.

### D. PHARMACEUTICAL COMPOSITIONS

As noted above, the compositions described herein (*e.g*., alphavirus replicon particles) may also include additional components, including, but not limited to, peptides, adjuvants, carriers, vehicles or other substances.

Thus, in certain embodiments, the compositions comprising alphavirus replicon particles are described herein may be administered in combination with one or more pharmaceutically acceptable salts, carriers, diluents, or recipients.

Pharmaceutically acceptable salts include, but are not limited to, mineral salts such as hydrochlorides, hydrobromides, phosphates, or sulfates, as well as salts of organic acids such as acetates, proprionates, malonates, or benzoates. Especially useful protein substrates are serum albumins, keyhole limpet hemocyanin, immunoglobulin molecules, thyroglobulin, ovalbumin, tetanus toxoid, and other proteins well known to those of skill in the art. Compositions of the invention can also contain liquids or excipients, such as water, saline, glycerol, dextrose, ethanol, or the like, singly or in combination, as well as substances such as wetting agents, emulsifying agents, or pH buffering agents. Liposomes can also be used as a carrier for a composition of the invention, such liposomes are described above.

In certain embodiments, the compositions include one or more polypeptides, for example one or more polypeptide antigens. The preparation of immunogenic compounds that contain immunogenic polypeptide(s) as active ingredients is known to those skilled in the art. Typically, such immunogenic compounds are prepared as injectables, either as liquid solutions or suspensions; solid forms suitable for solution in, or suspension in, liquid prior to injection can also be prepared. The preparation can also be emulsified, or the protein encapsulated in liposomes. Polypeptide antigens may be administered separately from the alphavirus replicon particles, for example prior to, concurrently or after administration of compositions comprising alphavirus replicon particles described above. The polypeptide antigens may be administered by any route, including mucosally, systemically or a combination thereof.

Similarly, one or more polynucleotides encoding one or more antigens may also be administered to the subject. Polynucleotides antigens may be administered separately from the alphavirus replicon particles, for example prior to, concurrently or after administration of compositions comprising alphavirus replicon particles described above. In other embodiments, the polynucleotide antigen(s) is(are) administered using pox (*e.g*., vaccinia) particles and/or adenovirus particles. *See, e.g.,* Doria-Rose et al. (2003) J. Virol. 77(21):11563-11577; Shayakhmetov et al. (2000) J. Virol. 74:2567-2583. The polynucleotides antigens may be administered by any route, including mucosally, systemically or a combination thereof. In certain embodiments, the polynucleotide comprises one or more alphavirus replicon vectors, each vector comprising at least one sequence encoding one or more antigenic polypeptides. In other embodiments, the polynucleotide comprises one or more pox virus vectors, including for example canary pox virus or vaccinia virus (e.g., Fisher-Hoch et al. (1989) Proc. Natl. Acad. Sci. USA 86:317-321; Flexner et al. (1989) Ann. N.Y. Acad. Sci. 569:86-103; Flexner et al. (1990) Vaccine 8:17-21; U.S. Patent Nos. 4,603,112; 4,769,330; and 5,017,487; International Publication No. WO 89/01973), each vector comprising at least one sequence encoding one or more antigenic polypeptides. In still other embodiments, the polynucleotide comprises one or more adenovirus virus vectors (*see, e.g.,* Berkner (1988) Biotechniques 6:616-627; Rosenfeld et al. (1991) Science 252:431-434), each vector comprising at least one sequence encoding one or more antigenic polypeptides.

Furthermore, as noted above, the mucosally- or systemically-administered immunogenic compositions may include one or more vehicles or carriers. Pharmaceutically acceptable carriers are well known to those in the art. Pharmaceutically acceptable carriers should not itself induce the production of antibodies harmful to the host. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Examples of particulate carriers include those derived from polymethyl methacrylate polymers, as well as microparticles derived from poly(lactides) and poly(lactide-co-glycolides), known as PLG. See, e.g., Jeffery et al. (1993) Pharm. Res. 10:362-368; McGee et al. (1997) J. Microencapsul. 14(2):197-210; O'Hagan et al. (1993) Vaccine 11(2):149-154. Such carriers are well known to those of ordinary skill in the art. Additionally, these carriers:may function as immunostimulating agents ("adjuvants").

One or more adjuvants may also be used in the compositions described herein. Adjuvants are substances that specifically or nonspecifically enhance the immune response to an antigen and include, for example, immunopotentiating molecules such as CpG oligos and imidazoquinoline compounds.

Examples of adjuvants that may be used in the compositions described herein include, but are not limited to, one or more of the following set forth below:

### A. OIL-EMULSIONS

Oil-emulsion compositions and formulations suitable for use as adjuvants in the invention (with or without other specific immunostimulating agents such as muramyl peptides or bacterial cell wall components) include squalene-water emulsions, such as MF59 (5% Squalene, 0.5% Tween 80, and 0.5% Span 85, formulated into submicron particles using a microfluidizer). See WO 90/14837. See also, Podda (2001) Vaccine 19: 2673-2680; Frey et al. (2003) Vaccine 21:4234-4237. MF59 is used as the adjuvant in the FLUAD™ influenza virus trivalent subunit vaccine.

Particularly preferred adjuvants for use in the compositions are submicron oil-in-water emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80™ (polyoxyethylenesorbitan monooleate), and/or 0.25-1.0% Span 85™ (sorbitan trioleate), and, optionally, N-acetylmuramyl-L-alanyl-D-isogluatminyl-L-alanine-2-(1'-2'-dipalmitoyl-*sn*-glycero-3-huydroxyphosphophoryloxy)-ethylamine (MTP-PE), for example, the submicron oil-in-water emulsion known as "MF59" (WO 90/14837; U.S. Patent No. 6,299,884; U.S. Patent No. 6,451,325; and Ott et al., "MF59 -- Design and Evaluation of a Safe and Potent Adjuvant for Human Vaccines" in Vaccine Design: The Subunit and Adjuvant Approach (Powell, M.F. and Newman, M.J. eds.) (New York: Plenum Press) 1995, pp. 277-296). MF59 contains 4-5% w/v Squalene (e.g. 4.3%), 0.25-0.5% w/v Tween 80™, and 0.5% w/v Span 85™ and optionally contains various amounts of MTP-PE, formulated into submicron particles using a microfluidizer such as Model 110Y microfluidizer (Microfluidics, Newton, MA). For example, MTP-PE may be present in an amount of about 0-500 µg/dose, more preferably 0-250 µg/dose and most preferably, 0-100 µg/dose. As used herein, the term "MF59-0" refers to the above submicron oil-in-water emulsion lacking MTP-PE, while the term MF59-MTP denotes a formulation that contains MTP-PE. For instance, "MF59-100" contains 100 µg MTP-PE per dose, and so on. MF69, another submicron oil-in-water emulsion for use herein, contains 4.3% w/v squalene, 0.25% w/v Tween 80™, and 0.75% w/v Span 85™ and optionally MTP-PE. Yet another submicron oil-in-water emulsion is MF75, also known as SAF, containing 10% squalene, 0.4% Tween 80™, 5% pluronic-blocked polymer L121, and thr-MDP, also microfluidized into a submicron emulsion. MF75-MTP denotes an MF75 formulation that includes MTP, such as from 100-400 µg MTP-PE per dose.

Submicron oil-in-water emulsions, methods of making the same and immunostimulating agents, such as muramyl peptides, for use in the compositions, are described in detail in WO 90/14837; U.S. Patent No. 6,299,884; and U.S. Patent No. 6,451,325.

Complete Freund's adjuvant (CFA) and incomplete Freund's adjuvant (IFA) may also be used as adjuvants in the invention.

### B. MINERAL CONTAINING COMPOSITIONS

Mineral containing compositions suitable for use as adjuvants in the invention include mineral salts, such as aluminum salts and calcium salts. The invention includes mineral salts such as hydroxides (*e.g*. oxyhydroxides), phosphates (*e.g*. hydroxyphosphates, orthophosphates), sulfates, *etc.* (see, *e.g.,* Vaccine Design: The Subunit and Adjuvant Approach (Powell, M.F. and Newman, M.J. eds.) (New York: Plenum Press) 1995, Chapters 8 and 9), or mixtures of different mineral compounds (*e.g*. a mixture of a phosphate and a hydroxide adjuvant, optionally with an excess of the phosphate), with the compounds taking any suitable form (*e.g*. gel, crystalline, amorphous, *etc.*)*,* and with adsorption to the salt(s) being preferred. The mineral containing compositions may also be formulated as a particle of metal salt (WO 00/23105).

Aluminum salts may be included in vaccines of the invention such that the dose of Al³⁺ is between 0.2 and 1.0 mg per dose.

In one embodiment the aluminum based adjuvant for use in the present invention is alum (aluminum potassium sulfate (AIK(SO₄)₂)), or an alum derivative, such as that formed in-situ by mixing an antigen in phosphate buffer with alum, followed by titration and precipitation with a base such as ammonium hydroxide or sodium hydroxide.

Another aluminum-based adjuvant for use in vaccine formulations of the present invention is aluminum hydroxide adjuvant (Al(OH)₃) or crystalline aluminum oxyhydroxide (AlOOH), which is an excellent adsorbant, having a surface area of approximately 500m²/g. Alternatively, aluminum phosphate adjuvant (AlPO₄) or aluminum hydroxyphosphate, which contains phosphate groups in place of some or all of the hydroxyl groups of aluminum hydroxide adjuvant is provided. Preferred aluminum phosphate adjuvants provided herein are amorphous and soluble in acidic, basic and neutral media.

In another embodiment the adjuvant of the invention comprises both aluminum phosphate and aluminum hydroxide. In a more particular embodiment thereof, the adjuvant has a greater amount of aluminum phosphate than aluminum hydroxide, such as a ratio of 2:1, 3:1, 4:1, 5:1, 6:1, 7:1, 8:1, 9:1 or greater than 9:1, by weight aluminum phosphate to aluminum hydroxide. More particular still, aluminum salts in the vaccine are present at 0.4 to 1.0 mg per vaccine dose, or 0.4 to 0.8 mg per vaccine dose, or 0.5 to 0.7 mg per vaccine dose, or about 0.6 mg per vaccine dose.

Generally, the preferred aluminum-based adjuvant(s), or ratio of multiple aluminum-based adjuvants, such as aluminum phosphate to aluminum hydroxide is selected by optimization of electrostatic attraction between molecules such that the antigen carries an opposite charge as the adjuvant at the desired pH. For example, aluminum phosphate adjuvant (iep = 4) adsorbs lysozyme, but not albumin at pH 7.4. Should albumin be the target, aluminum hydroxide adjuvant would be selected (iep 11.4). Alternatively, pretreatment of aluminum hydroxide with phosphate lowers its isoelectric point, making it a preferred adjuvant for more basic antigens.

### C. SAPONIN FORMULATIONS

Saponin formulations are also suitable for use as adjuvants in the invention. Saponins are a heterologous group of sterol glycosides and triterpenoid glycosides that are found in the bark, leaves, stems, roots and even flowers of a wide range of plant species. Saponins isolated from the bark of the *Quillaia saponaria* Molina tree have been widely studied as adjuvants. Saponins can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. Saponin adjuvant formulations include STIMULON^{®} adjuvant (Antigenics, Inc., Lexington, MA).

Saponin compositions have been purified using High Performance Thin Layer Chromatography (HP-TLC) and Reversed Phase High Performance Liquid Chromatography (RP-HPLC). Specific purified fractions using these techniques have been identified, including QS7, QS17, QS18, QS21, QH-A, QH-B and QH-C. Preferably, the saponin is QS21. A method of production of QS21 is disclosed in U.S. Patent No. 5,057,540. Saponin formulations may also comprise a sterol, such as cholesterol (see WO 96/33739).

Combinations of saponins and cholesterols can be used to form unique particles called Immunostimulating Complexes (ISCOMs). ISCOMs typically also include a phospholipid such as phosphatidylethanolamine or phosphatidylcholine. Any known saponin can be used in ISCOMs. Preferably, the ISCOM includes one or more of Quil A, QHA and QHC. ISCOMs are further described in EP 0 109 942, WO 96/11711 and WO 96/33739. Optionally, the ISCOMS may be devoid of (an) additional detergent(s). See WO 00/07621.

A review of the development of saponin based adjuvants can be found in Barr et al. (1998) Adv. Drug Del. Rev. 32:247-271. See also Sjolander et al. (1998) Adv. Drug Del. Rev. 32:321-338.

### D. VIROSOMES AND VIRUS LIKE PARTICLES (VLPS)

Virosomes and Virus Like Particles (VLPs) are also suitable as adjuvants for use in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in WO 03/024480; WO 03/024481; Niikura et al. (2002) Virology 293:273-280; Lenz et al. (2001) J. Immunol. 166(9):5346-5355; Pinto et al. (2003) J. Infect. Dis. 188:327-338; and Gerber et al. (2001) J. Virol. 75(10):4752-4760. Virosomes are discussed further in, for example, Gluck et al. (2002) Vaccine 20:B 10-B 16. Immunopotentiating reconstituted influenza virosomes (IRIV) are used as the subunit antigen delivery system in the intranasal trivalent INFLEXAL™ product (Mischler and Metcalfe (2002) Vaccine 20 Suppl 5:B17-B23) and the INFLUVAC PLUS™ product.

### E. BACTERIAL OR MICROBIAL DERIVATIVES

Adjuvants suitable for use in the invention include bacterial or microbial derivatives such as:

(1) Non-toxic derivatives of enterobacterial lipopolysaccharide (LPS): Such derivatives include Monophosphoryl lipid A (MPL) and 3-O-deacylated MPL (3dMPL). 3dMPL is a mixture of 3 De-O-acylated monophosphoryl lipid A with 4, 5 or 6 acylated chains. A preferred "small particle" form of 3 De-O-acylated monophosphoryl lipid A is disclosed in EP 0 689 454. Such "small particles" of 3dMPL are small enough to be sterile filtered through a 0.22 micron membrane (see EP 0 689 454). Other non-toxic LPS derivatives include monophosphoryl lipid A mimics, such as aminoalkyl glucosaminide phosphate derivatives *e.g.* RC-529. See Johnson et al. (1999) Bioorg. Med. Chem. Lett. 9:2273-2278.

(2) Lipid A Derivatives: Lipid A derivatives include derivatives of lipid A from *Escherichia coli* such as OM-174. OM-174 is described for example in Meraldi et al. (2003) Vaccine 21:2485-2491; and Pajak et al. (2003) Vaccine 21:836-842.

(3) Immunostimulatory oligonucleotides: Immunostimulatory oligonucleotides or polymeric molecules suitable for use as adjuvants in the invention include nucleotide sequences containing a CpG motif (a sequence containing an unmethylated cytosine followed by guanosine and linked by a phosphate bond). Bacterial double stranded RNA or oligonucleotides containing palindromic or poly(dG) sequences have also been shown to be immunostimulatory. The CpG's can include nucleotide modifications/analogs such as phosphorothioate modifications and can be doublestranded or single-stranded. Optionally, the guanosine may be replaced with an analog such as 2'-deoxy-7-deazaguanosine. See Kandimalla et al. (2003) Nucl. Acids Res. 31(9): 2393-2400; WO 02/26757; and WO 99/62923 for examples of possible analog substitutions. The adjuvant effect of CpG oligonucleotides is further discussed in Krieg (2003) Nat. Med. 9(7):831-835; McCluskie et al. (2002) FEMS Immunol. Med. Microbiol. 32:179-185; WO 98/40100; U.S. Patent No. 6,207,646; U.S. Patent No. 6,239,116; and U.S. Patent No. 6,429,199.

The CpG sequence may be directed to TLR9, such as the motif GTCGTT or TTCGTT. See Kandimalla et al. (2003) Biochem. Soc. Trans. 31 (part 3):654-658. The CpG sequence may be specific for inducing a Th1 immune response, such as a CpG-A ODN, or it may be more specific for inducing a B cell response, such a CpG-B ODN. CpG-A and CpG-B ODNs are discussed in Blackwell et al. (2003) J. Immunol. 170(8):4061-4068; Krieg (2002) TRENDS Immunol. 23(2): 64-65; and WO 01/95935. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, Kandimalla et al. (2003) BBRC 306:948-953; Kandimalla et al. (2003) Biochem. Soc. Trans. 31(part 3):664-658; Bhagat et al. (2003) BBRC 300:853-861; and WO03/035836.

Immunostimulatory oligonucleotides and polymeric molecules also include alternative polymer backbone structures such as, but not limited to, polyvinyl backbones (Pitha et al. (1970) Biochem. Biophys. Acta 204(1):39-48; Pitha et al. (1970) Biopolymers 9(8):965-977), and morpholino backbones (U.S. Patent No. 5,142,047; U.S. Patent No. 5,185,444). A variety of other charged and uncharged polynucleotide analogs are known in the art. Numerous backbone modifications are known in the art, including, but not limited to, uncharged linkages (e.g., methyl phosphonates, phosphotriesters, phosphoamidates, and carbamates) and charged linkages (e.g., phosphorothioates and phosphorodithioates).

(4) ADP-ribosylating toxins and detoxified derivatives thereof: Bacterial ADP-ribosylating toxins and detoxified derivatives thereof may be used as adjuvants in the invention. Preferably, the protein is derived *from E. coli* (i.e., *E. coli* heat labile enterotoxin "LT"), cholera ("CT"), or pertussis ("PT"). The use of detoxified ADP-ribosylating toxins as mucosal adjuvants is described in WO 95/17211 and as parenteral adjuvants in WO 98/42375. Preferably, the adjuvant is a detoxified LT mutant such as LT-K63, LT-R72, and LTR192G. The use of ADP-ribosylating toxins and detoxified derivatives thereof, particularly LT-K63 and LT-R72, as adjuvants can be found in the following references: Beignon et al. (2002) Infect. Immun. 70(6):3012-3019; Pizza et al. (2001) Vaccine 19:2534-2541; Pizza et al. (2000) Int. J. Med. Microbiol. 290(4-5):455-461; Scharton-Kersten et al. (2000) Infect. Immun. 68(9):5306-5313; Ryan et al. (1999) Infect. Immun. 67(12):6270-6280; Partidos et al. (1999) Immunol. Lett. 67(3):209-216; Peppoloni et al. (2003) Vaccines 2(2):285-293; and Pine et al. (2002) J. Control Release 85(1-3):263-270. Numerical reference for amino acid substitutions is preferably based on the alignments of the A and B subunits of ADP-ribosylating toxins set forth in Domenighini et al. (1995) Mol. Microbiol. 15(6):1165-1167.

### F. BIOADHESIVES AND MUCOADHESIVES

Bioadhesives and mucoadhesives may also be used as adjuvants in the invention. Suitable bioadhesives include esterified hyaluronic acid microspheres (Singh et al. (2001) J. Cont. Release 70:267-276) or mucoadhesives such as cross-linked derivatives of polyacrylic acid, polyvinyl alcohol, polyvinyl pyrollidone, polysaccharides and carboxymethylcellulose. Chitosan and derivatives thereof may also be used as adjuvants in the invention (see WO 99/27960).

### G. MICROPARTICLES

Microparticles may also be used as adjuvants in the invention. Microparticles (i.e. a particle of∼100nm to∼150µm in diameter, more preferably -200nm to ∼30 µm in diameter, and most preferably ∼500 nm to ∼10 µm in diameter) formed from materials that are biodegradable and non-toxic (e.g. a poly(α-hydroxy acid), a polyhydroxybutyric acid, a polyorthoester, a polyanhydride, a polycaprolactone, *etc*.), with poly(lactide-co-glycolide) are preferred, optionally treated to have a negatively-charged surface (e.g. with SDS) or a positively-charged surface (e.g. with a cationic detergent, such as CTAB).

### H. LIPOSOMES

Examples of liposome formulations suitable for use as adjuvants in the invention are described in U.S. Patent No. 6,090,406; U.S. Patent No. 5,916,588; and EP 0 626 169.

### I. POLYOXYETHYLENE ETHER AND POLYOXYETHYLENE ESTER FORMULATIONS

Adjuvants suitable for use in the invention include polyoxyethylene ethers and polyoxyethylene esters (see, e.g., WO 99/52549). Such formulations further include polyoxyethylene sorbitan ester surfactants in combination with an octoxynol (WO 01/21207) as well as polyoxyethylene alkyl ethers or ester surfactants in combination with at least one additional non-ionic surfactant such as an octoxynol (WO 01/21152).

Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### J. POLYPHOSPHAZENE (PCPP)

PCPP formulations suitable for use as adjuvants in the invention are described, for example, in Andrianov et al. (1998) Biomaterials 19(1-3):109-115; and Payne et al. (1998) Adv. Drug Del. Rev. 31(3):185-196*.*

### K. MURAMYL PEPTIDES

Examples of muramyl peptides suitable for use as adjuvants in the invention include N-acetyl-muramyl-L-threonyl-D-isoglutamine (thr-MDP), N-acetyl-normuramyl-l-alanyl-d-isoglutamine (nor-MDP), and N-acetylmuramyl-l-alanyl-d-isoglutaminyl-l-alanine-2-(1'-2'-dipalmitoyl-sn-glycero-3-hydroxyphosphoryloxy)-ethylamine MTP-PE).

### L. IMIDAZOQUINOLINE COMPOUNDS

Examples of imidazoquinoline compounds suitable for use as adjuvants in the invention include Imiquimod and its analogues, which are described further in Stanley (2002) Clin. Exp. Dermatol. 27(7):571-577; Jones (2003) Curr. Opin. Investig. Drugs 4(2):214-218; and U.S. Patent Nos. 4,689,338; 5,389,640; 5,268,376; 4,929,624; 5,266,575; 5,352,784; 5,494,916; 5,482,936; 5,346,905; 5,395,937; 5,238,944; and 5,525,612.

Examples of thiosemicarbazone compounds suitable for use as adjuvants in the invention, as well as methods of formulating, manufacturing, and screening for such compounds, include those described in WO 04/60308. The thiosemicarbazones are particularly effective in the stimulation of human peripheral blood mononuclear cells for the production of cytokines, such as TNF- .

### N. TRYPTANTHRIN COMPOUNDS

Examples of tryptanthrin compounds suitable for use as adjuvants in the invention, as well as methods of formulating, manufacturing, and screening for such compounds, include those described in WO 04/64759. The tryptanthrin compounds are particularly effective in the stimulation of human peripheral blood mononuclear cells for the production of cytokines, such as TNF- .

The invention may also comprise combinations of aspects of one or more of the adjuvants identified above. For example, the following adjuvant compositions may be used in the invention:

(1) a saponin and an oil-in-water emulsion (WO 99/11241);

(2) a saponin (e.g., QS21) + a non-toxic LPS derivative (e.g. 3dMPL) (see WO 94/00153);

(3) a saponin (e.g., QS21) + a non-toxic LPS derivative (e.g. 3dMPL) + a cholesterol;

(4) a saponin (e.g., QS21) + 3dMPL + IL-12 (optionally + a sterol) (WO 98/57659);

(5) combinations of 3dMPL with, for example, QS21 and/or oil-in-water emulsions (see EP 0 835 318; EP 0 735 898; and EP 0 761 231);

(6) SAF, containing 10% Squalane, 0.4% Tween 80, 5% pluronic-block polymer L121, and thr-MDP, either microfluidized into a submicron emulsion or vortexed to generate a larger particle size emulsion;

(7) Ribi^{™} adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™);

(8) one or more mineral salts (such as an aluminum salt) + a non-toxic derivative of LPS (such as 3dPML);

(9) one or more mineral salts (such as an aluminum salt) + an immunostimulatory oligonucleotide (such as a nucleotide sequence including a CpG motif).

### O. HUMAN IMMUNOMODULATORS

Human immunomodulators suitable for use as adjuvants in the invention include cytokines, such as interleukins (e.g. IL-1, IL-2, IL-4, IL-5, IL-6, IL-7, IL-12, etc.), interferons (e.g. interferon-y), macrophage colony stimulating factor (M-CSF), and tumor necrosis factor (TNF).

Aluminum salts and MF59 are preferred adjuvants for use with injectable influenza vaccines. Bacterial toxins and bioadhesives are preferred adjuvants for use with mucosally-delivered vaccines, such as nasal vaccines.

For instance, in certain embodiments, the mucosally administered immunogenic composition further comprises a mucosal adjuvant. Suitable adjuvants include: CpG containing oligo, bioadhesive polymers, see International Publication No. WO 99/62546 and WO 00/50078; E. *coli* heat-labile entertoxin ("LT") or detoxified mutants thereof or cholera toxin ("CT") or detoxified mutant thereof or microparticles that are formed from materials that are biodegradeable and non-toxic. Preferred LT mutants include K63 or R72. See, e.g., International Publication Nos. WO 93/13202; WO 95/017211; WO 97/02348; and WO 97/29771; European Patent Nos. EP 0 620 850 B1; EP 0 869 181 B1; and EP 0 732 937 B1.

The compositions may also be lyophilized or otherwise made storage-stable.

The compositions preferably comprise a "therapeutically effective amount" of the macromolecule of interest. That is, an amount of alphavirus replicon particles/polypeptide antigen/polynucleotide vector/etc. will be included in the compositions that will cause the subject to produce a sufficient response, in order to prevent, reduce, eliminate or diagnose symptoms. The exact amount necessary will vary, depending on the subject being treated; the age and general condition of the subj ect to be treated; the severity of the condition being treated; in the case of an immunological response, the capacity of the subject's immune system to synthesize antibodies; the degree of protection desired and the particular antigen selected and its mode of administration, among other factors. An appropriate effective amount can be readily determined by one of skill in the art. Thus, a " therapeutically effective amount" will fall in a relatively broad range that can be determined through routine trials.

For example, for purposes of the present invention, the titers of the alphavirus replicon particles in the compositions described herein is preferably above 10⁶ IU (infectious units) per dose, more preferably above about 10⁷ IU per dose and even more preferably at least about 10⁸IU per dose. Dosages for polypeptide antigens will vary from microgram to milligram amounts or even higher, for example from about 1 µg/dose to about 10 mg/dose (including any amount therebetween), preferably between about 100 µg and about 5 mg/dose (including any amount therebetween)" and more preferably between about 250 µg and 750 µg/dose (including any amount therebetween). In certain embodiments, approximately 0.5 mg of protein is administered per dose.

### E. KITs

Also described herein are kits for inducing and augmenting a mucosal immune response to a target antigen. Such a kit preferably comprises a priming amount of an alphavirus replicon particle containing nucleic acids encoding one or more antigens useful for priming the immune response to a target antigen or an immunologically active fragment thereof. Also included in the kits is an effective amount of a boosting vaccine composition comprising an alphavirus replicon particle containing nucleic acids encoding one or more antigens useful for boosting the immune response to a target antigen or an immunologically active fragment thereof. The kit can comprise single or multiple doses of the priming composition, of the boosting composition or of both priming and boosting compositions. Thus, in a particular embodiment, to facilitate repeat administrations, the kit can comprise a plurality of vials for one or both compositions, each vial comprising the dose to be administered to the subject at each administration.

Other components of the kit include applicators for administering each composition. By the term "applicator" as the term is used herein, is meant any device including but not limited to a hypodermic syringe, gene gun, nebulizer, dropper, bronchoscope, suppository, impregnated or coated vaginally-insertable material such as a tampon, douche preparation, solution for vaginal irrigation, retention enema preparation, suppository, or solution for rectal or colonic irrigation for applying the priming and/or the boosting compositions either systemically or mucosally, respectively, to the human or veterinary patient.

Still another component involves instructions for using the kit. The instructions for using the kit depend on the antigen for which the kit is to be used. The kit may also include instructions on how to apply the priming and/or boosting composition, e.g., using the applicator provided therewith. As one example, in the case of mucosal immunity employing the compositions of the examples below, the instructions comprise directions on how to administer the priming DNA vaccine composition mucosally (e.g., intranasally) and how to subsequently administer the boosting composition systemically (e.g., intramuscularly).

The following example is offered by way of illustration, and not by way of limitation.

### EXAMPLE 1

### ENHANCED ANTI-HIV-ENV ANTIBODY RESPONSES IN RHESUS MACAQUES FOLLOWING COMBINATIONS OF MUCOSAL AND SYSTEMIC IMMUNIZATIONS WITH CHIMERIC ALPHAVIRUS BASED

### REPLICON PARTICLES

The human immunodeficiency virus (HIV) continues to cause significant infections in the world, leading to acquired immunodeficiency syndrome. Most HIV transmissions occur through the vaginal mucosa.

Intranasal (IN) immunization has been shown to induce local immunity not only in the nasal associated lymphoid tissue and the lung, but also in the female genital tract in rodents (Vajdy et al. (2001) J. Infect. Dis. 184:1613-1616; Gupta et al. (2005) J. Virol. 79:7135-7145; Wu et al. (1992) Infect. Immun. 61:314-322; Giuliani et al. (1998) J. Exp. Med. 187:1123-1132; Ugozzoli et al. (1998) Immunology 93:563-571; Asanuma et al. (1998) Vaccine 16:1257-1262; Lowell et al. (1997) J. Infect. Dis. 175:292-301) as well as in humans and nonhuman primates (Russell et al. (1996) Infect. Immunity 64:1272-1283; Imaoka et al. (1998) J.Immunol. 161:5952-5958; Moldoveanu et al. (1995) Vaccine 13:1006-1012; Bergquist et al. (1997) Infect. Immun. 65:2676-2684). Similarly, intra-vaginal and intra-rectal immunizations of rodents and humans also induce local immunity in the genital tract. *See, e.g.,* Vajdy et al. (2001) J. Infect. Dis. 184:1613-1616; Kozlowski et al. (1999) J. Infect. Dis. 179 Suppl 3:S493-S498; Eriksson et al. (1998) Infect. Immun. 66:5889-5896).

For instance, VEE-derived and chimera replicon particles, with VEE-derived replicon RNA and SIN-derived surface glycoproteins (VEE/SIN), expressing HIV-1 gag antigen induced significantly higher systemic cell mediated responses than the previously described SIN replicon particles following systemic (IM) immunization of mice with the vectors (Perri et al. (2003) J. Virol. 77:10394-10493). In addition, mucosal or systemic immunizations with VEE/SIN expressing HIV-gag (VEE/SIN-gag) resulted in enhanced cellular IFNy responses and better protection against vaginal challenge with vaccinia virus expressing HIV-gag (Gupta et al. (2005) J. Virol. 79:7135-7145). Furthermore, mucosal followed by systemic immunizations with protein antigens and adjuvants induced have been shown to enhance mucosal and systemic immune responses compared to systemic alone or mucosal alone immunizations (Vajdy et al. (2003) Immunol. 110:86-94; Vajdy et al. (2004) AIDS Research and Human Retroviruses 20:1269-1281).

Thus, in order to determine the immunogenicity of the replicon particles using a mucosal prime(s)-systemic boost primate model, the following experiments were performed.

### A. Materials and Methods

### 1. Preparation of alphavirus replicon particles

The VEE/SIN-Env gp140 and SIN-Env gp140 or green fluorescent protein (GFP) replicon particles were prepared as described previously in Perri et al. (2003) J. Virol. 77:10394-10493. Briefly, replicon particles were generated by co-transfection of in vitro-transcribed RNA species corresponding to a replicon and two defective helpers, with one helper expressing capsid protein and the other expressing envelope glycoproteins E2 and E1, as described in Polo et al. (1999) Proc. Natl. Acad. Sci. USA 96:4598-4603. Titers of replicon particles on BHK-21 cells were determined as described in Perri et al. (2000) J. Virol. 74:9802-9807.

Replicon particles expressing HIV Env gp140 or GFP were harvested as culture supernatants at 20 and 30 hours post transfection, clarified by filtration, and purified by cation exchange chromatography. Replicon particle titers as infectious units (IU) per ml were determined by intracellular staining of expressed gp140 or GFP, following overnight infection of BHK-21 cells with serial dilutions of particles.

### 2. Animals and immunizations

Four juvenile, female, rhesus macaques (Macaca mulatta) were immunized intranasally (INS) 3 times with 10⁸ (infectious units) IU of VEE/SIN or SIN replicon particles encoding HIV-gp140 per group, at 4 weeks intervals and then both groups were immunized intramuscularly (IM) with 10⁸ IUVEE/SIN. In addition, four juvenile, female, rhesus macaques were immunized intramuscularly 2 times with 1 mg PLG-DNA encoding Env gp140 at 4 week intervals and then immunized intramuscularly with 10⁸ IU VEE/SIN. Optionally, the groups were also immunized intramuscularly 2 times with oligomeric gp140 (o-gp140 protein), as shown below (Study Design #2):

| **STUDY DESIGN #2** | | |
|---|---|---|
| **PRIME** | **BOOST** | **BOOST** |
| 1. VEE/STN INx3 | VEE/SIN IMx2 | o-gp140 protein IMx2 |
| 2. SIN INx3 | VEE/SIN IMx2 | o-gp140 protein IMx2 |
| 3. PLG-DNA IMx2 | VEE/SIN IMx2 | o-gp140 protein IMx2 |

Peripheral blood mononuclear cells (PBMC) were collected before the immunizations, and serum, vaginal wash and saliva samples were collected at 2 weeks after each immunization.

All animals were housed at the University of California at Davis Primate Research Center in accordance with standards of the Association for Assessment and Accreditation of Laboratory Animal Care and University of California at Davis's Animal Care and Use Committee. The investigators adhered to the "Guide for the Care and Use of Laboratory Animals" prepared by the Committee on Care and Use of Laboratory Animals of the Institute of Laboratory Resources, National Research Council.

### 3. Collection of samples

The macaques in all groups were bled before and 2 weeks after each immunization under anesthesia. A total of 30 ml of heparinized whole blood was collected. Peripheral blood mononuclear cells (PBMC) were isolated by Ficol separation using standard procedures and used either immediately in an ELISPOT assay or frozen for later use. Plasma was collected from the same 30 ml of whole blood and stored at -20°C. Moreover, approximately 1 ml vaginal lavage or saliva were collected from each macaque under anesthesia, frozen immediately on dry ice and stored at - 80°C.

### 4. ELISA Assays

HIV-1 o-gp140 env-specific serum IgG titers were quantified by a standard ELISA assay, as previously described in Vajdy et al. (2004) AIDS Res Hum Retroviruses 20(11):1269-81 and Vajdy et al. (2004) Immunol Cell Biol. 82(6):617-27. Briefly, ELISA plates (96 well U bottom by Nunc Maxisorp) were coated with p55 or o-gp140 protein at 5 mg/ml. After washing with 1X PBS + 0.03% Tween 20 (Sigma), the wells were blocked and serially diluted,samples were added in an assay diluent made up of 1X PBS + 5% goat serum (Gibco Brl) + 0.03% Tween 20 (Sigma). A serum standard was included in each assay for quantitation purposes. The samples and standard sera were incubated at 37°C for one hour and washed with PBS/0.03% Tween. The samples were then incubated at 1:40,000 dilution of a goat anti-mouse IgG-HRP (Caltag), developed with tetramethylbenzidine (TMB-Kirkegaard and Perry) for 15 minutes, and stopped with 2N MHC1. The optical density of each well was measured using Titertek at 450 nm.

Vaginal lavage and saliva samples were assayed for total and gp140-specific IgA and IgG using a bioluminescent immunosorbant assay (BIA) as previously described in Giulani et al. (1998) J. Exp. Med. 187:1123-1132 and Vajdy et a1. (2004) AIDS Res Hum Retroviruses 20(11):1269-81 and Vajdy et al. (2004) Immunol Cell Biol. 82(6):617-27. Briefly, ELISA plates (MicroLite obtained from Dynatech) were first coated with the o-gp140 antigen (5 mg/ml) or goat anti-rhesus IgA or IgG overnight. After blocking (5% goat serum, 25 mM Tris, 10 mM EGTA, 150 mM KC1, 2 mg/ml BSA, 0.3% Tween-20, pH 7.5), plates were added 1:3 serially diluted vaginal wash samples in blocking buffer. The plates were developed using 1:1000 diluted goat anti-rhesus macaque IgA or IgG biotin conjugate (from a different source than the coating antibodies).

Plates were then incubated with 1:500 diluted streptavidin-jellyfish aequorin conjugate (SeaLite Sciences, Bogart, GA). Luminescence was triggered with 10 mM calcium acetate and measured using a luminometer (Dynatech ML3000). Quantitation was based on relative light units (RLU) representing total luminescence integrated over three seconds (arbitrary units). Titers represent log dilution values linearly extrapolated from the log RLU data to a cutoff value at least two standard deviations above mean background.

### 5. Ex vivo infection of Rhesus macaque PBMC with VEE, SIN or VEE/SIN

Peripheral blood mononuclear cells (PBMC) were prepared from naïve rhesus macaques. The cells were re-suspended at 20 X 10⁶/ml in 1% RPMI in 50 µl and then were infected with 250 µl of VEE or 500 µl of VEE/SIN or SIN each expressing green fluorescent gene (GFP) at multiplicity of infection (MOI) of 1:100. The cells and the particles were then incubated for 90 minutes with shaking at 37°C and transferred to 24 well plates and incubated overnight at 37°C. BHK cells were used as positive controls. The next day the cells were surface stained with mouse antihuman CD 14, CD 11b and CD20 (Pharmingen, San Diego, California) and analyzed by FACS.

### 6. Statistical Analysis

Statistical analysis was performed using Student's T test available on Microsoft Excel software. An F-test was first performed to determine whether the variances of the individual log values between the two groups was equal, and then a Student's t test (two tail, two sample assuming equal variances) was performed for 95% confidence intervals.

### B. Results

### 1. Antibody Responses: IgG

Female rhesus macaques immunized as described above.

### 1.A. VEE/SIN v. SIN replicon particles

Following 3 IN immunizations, 3 out of 4 macaques immunized with VEE/SIN seroconverted, whereas 2 out of 4 macaques immunized with SIN seroconverted, although no significant differences could be discerned between the two groups (Table 1). Interestingly, 1N immunizations with VEE/SIN chimeric replicon particles induced higher vaginal and saliva anti-HIV-env IgG responses compared to IN immunizations with SIN (Table 1).

As shown in Tables 1 and 2, anti-gp140 serum IgG responses were induced after intra-nasal priming immunizations with SIN or VEE/SIN, and were enhanced after intra-muscular boosting immunizations with VEE/SIN. Further, anti-gp 140 vaginal IgG responses were induced after intra-nasal priming immunizations with SIN or VEE/SIN, but were not enhanced after intra-muscular boosting immunizations with VEE/SIN.

**Table 1**

| Serum anti-gp140 IgA titers | | Serum anti-gp140 IgG titers | |
|---|---|---|---|
| VEE/SIN INX3 (2WP3) | SIN INx3 (2WP3) | VEE/SIN INX3 (2WP3) | SIN INx3 (2WP3) |
| 5 | 5 | 21 | 162 |
| 5 | 5 | 5 | 5 |
| 5 | 5 | 118 | 5 |
| 6201 | 5 | 84 | 15 |
| | Mean ± SEM | | |
| N=4 | N=4 | 57.00 ± 26.54 N=4 | 46.75 ± 38.49 N=4 |
| **P>0.05** | | **P=>0.05** | |

| Vaginal anti-gp140 IgA titers / Total IgA titers | | Vaginal anti-gp140 IgG titers / Total IgG titers | |
|---|---|---|---|
| VEE/SIN INX3 (2WP3) | SIN INx3 (2WP3) | VEE/SIN INX3 (2WP3) | SIN INx3 (2WP3) |
| 0.98 | 0.17 | 5.71 | 0.06 |
| 0.50 | 0.48 | 5.02 | 0.42 |
| 0.14 | 0.20 | 7.13 | 1.10 |
| 0.57 | 0.03 | 6.34 | 0.44 |
| | Mean ± SEM | | |
| 0.5475 ± 0.1722 N=4 | 0.2200 ± 0.09425 N=4 | 6.050 ± 0.4497 N=4 | 0.5050 ± 0.2167 N=4 |
| **P>0.05** | | **P=0.0001** | |

| saliva anti-gp140 IgA titers / Total IgA titers | | Saliva anti-gp140 IgG titers / Total IgG titers | |
|---|---|---|---|
| VEE/SIN INx3 (2WP3) | SIN INx3 (2WP3) | VEE/SIN INX3 (2WP3) | SIN lNx3 (2WP3) |
| 0.48 | 0.23 | 0.78 | 0.44 |
| 0.21 | 3.02 | 0.39 | 0.76 |
| 0.50 | 0.21 | 0.78 | 0.67 |
| 1.26 | 0.18 | 0.15 | 0.61 |
| | Mean ± SEM | | |
| 0.6125 ± 0.2257 N=4 | 0.9100 ± 0.7034 N=4 | 0.5250 ± 0.1552 N=4 | 0.6200 ± 0.06745 N=4 |
| **P>0.05** | | **P>0.05** | |

### 1.B. Study Design #2

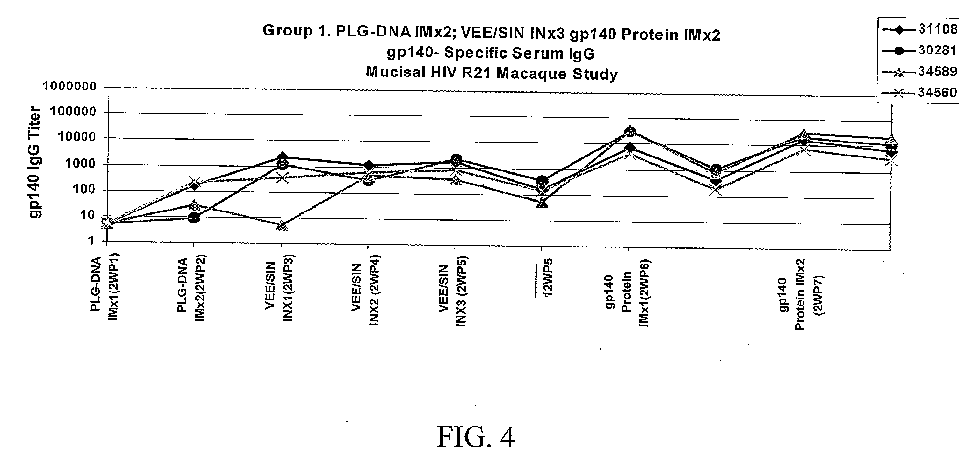

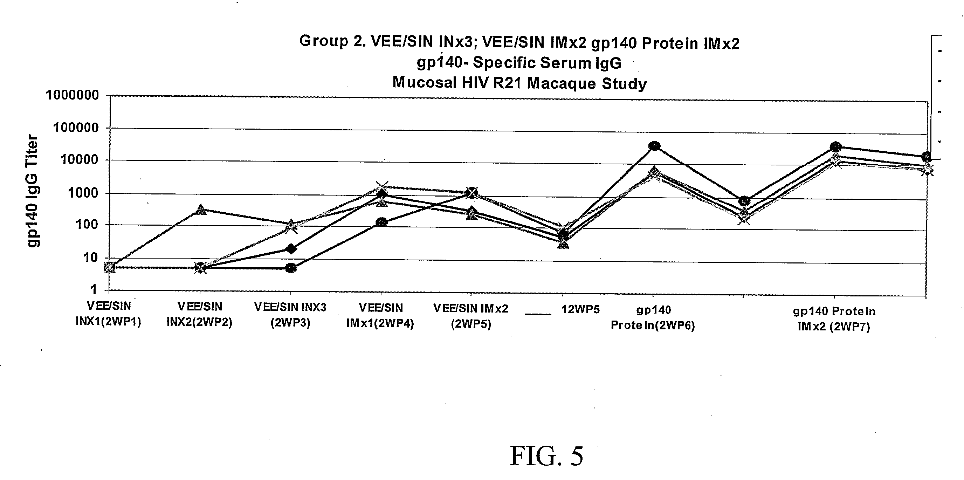

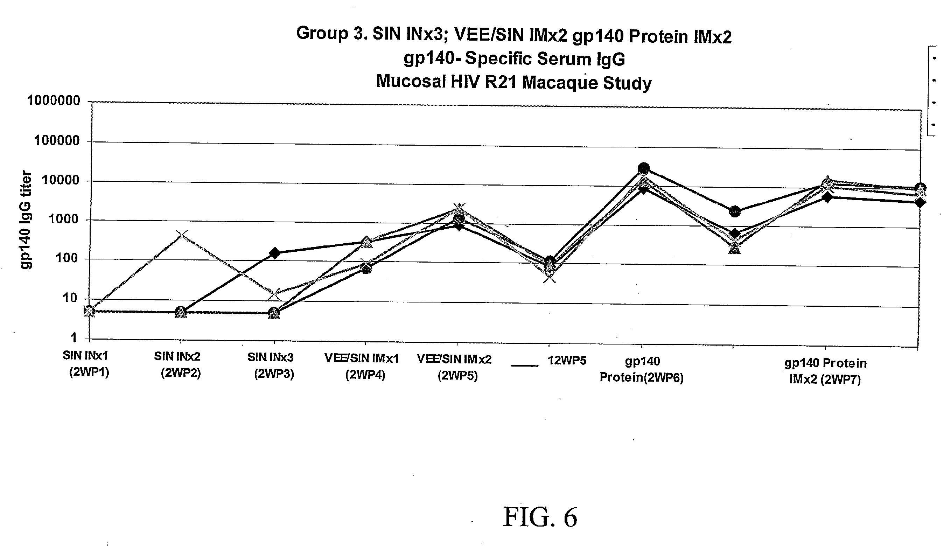

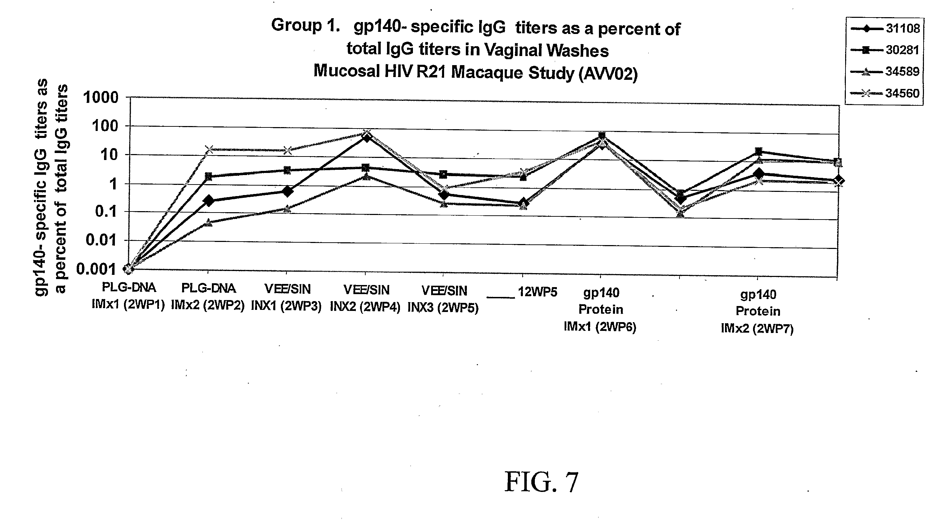

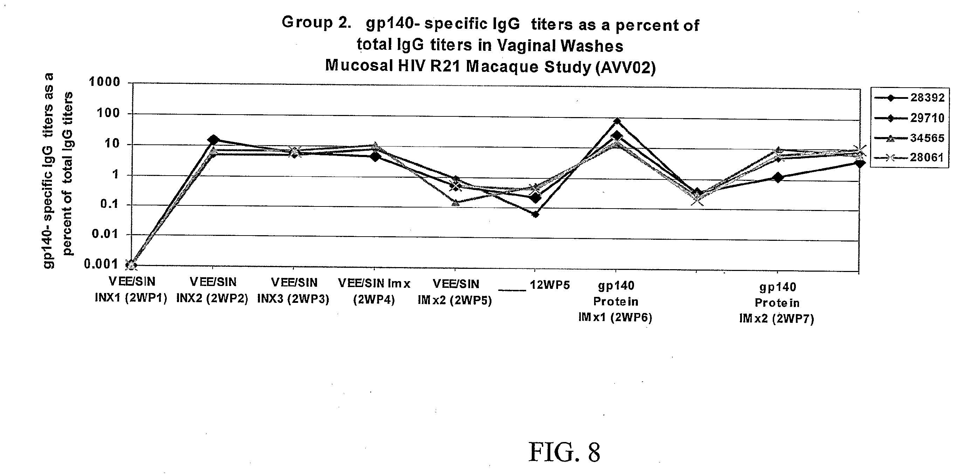

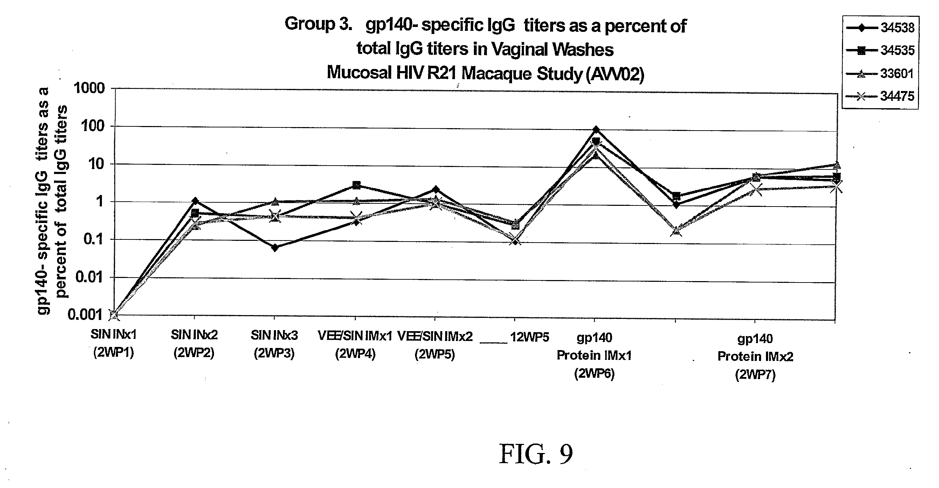

In addition, IgG titers from the groups receiving additional protein boost was also determined from serum (FIGs. 4, 5 and 6) and vaginal washes (FIGs. 7, 8 and 9).

These results show that anti-gp140 serum IgG responses were significantly enhanced after intra-muscular boosting immunizations with o-gp140 protein in MF59 following priming IN/IM immunizations with replicon particles. Similarly, the anti-gp 140 vaginal IgG responses were significantly enhanced after intra-muscular boosting immunizations with o-gp140 protein in MF59 following priming IN/IM immunizations with replicon particles.

### 2. Antibody Responses: IgA

### 2.A. VEE/SIN v. SIN replicon particles

In addition, following 3 intra-nasal (IN) immunizations with VEE/SIN vs. SIN replicon particles and one subsequent intra-muscular immunization with VEE/SIN particles, the antibody responses were significantly enhanced in both groups. Macaques previously immunized IN with VEE/SIN replicon particles had significantly higher serum as well as vaginal and saliva IgG antibody responses compared to the macaques previously immunized IN with SIN particles (Table 2). In particular, anti-gp140 serum IgA responses were induced after intra-nasal priming immunizations with SIN or VEE/SIN, and were enhanced after intra-muscular boosting immunizations with VEE/SIN. Furthermore, anti-gp140 vaginal and saliva IgA responses were induced after intra-nasal priming immunizations with SIN or VEE/SIN, but were not enhanced after intra-muscular boosting immunizations with VEE/SIN.

**Table 2**

| Serum anti-gp140 IgA titers | | Serum anti-gp140 IgG titers | |
|---|---|---|---|
| VEE/SIN INX3, IMx1 | | VEE/SIN INX3, IMx1 | |
| (2WP4) | SIN INx3, IMx1 (2WP4) | (2WP4) | SIN INx3, IMx1 (2WP4) |
| 11470 | 5 | 1010.5 | 324.5 |
| 5 | 7691 | 130 | 68.5 |
| 5 | 5 | 636 | 319 |
| 16717 | 5 | 1700 | 90.5 |
| | Mean ± SEM | | |
| 7049 ± 4206 N=4 | 1927 ± 1922 N=4 | 1116 ± 311.6 N=3 | 244.7 ± 77.10 N=3 |
| **P>0.05** | | **P=0.049** | |

| Vaginal anti-gp140 IgA titers/ Total IgA titers | | Vaginal anti-gp140 IgG titers/ Total IgG titers | |
|---|---|---|---|
| VEE/SIN INX3, IMx1 | | VEE/SIN INX3, IMx1 | |
| (2WP4) | SIN INx3, IMx1 (2WP4) | (2WP4) | SIN lNx3, IMx1 (2WP4) |
| 0.78 | 0.78 | 4.82 | 0.33 |
| 1.42 | 8.06 | 8.26 | 3.13 |
| 0.18 | 2.34 | 11.47 | 1.13 |
| 1.05 | 1.27 | | 0.42 |
| | Mean ± SEM | | |
| 0.8575 ± 0.2612 N=4 | 3.113 ± 1.681 N=4 | 8.183 ± 1.920 N=3 | 1.253 ± 0.6509 N=4 |
| **P>0.05** | | **P=0.009** | |

| Saliva anti-gp140 IgA titers/ Total IgA titers | | Saliva anti-gp140 IgG titers / Total IgG titers | |
|---|---|---|---|
| VEE/SIN INX3, IMx1 | | VEE/SIN INX3, IMx1 | |
| (2WP4) | SIN INx3, IMx1 (2WP4) | (2WP4) | SIN INx3, IMx1 (2WP4) |
| 16.10 | 0.99 | 1.04 | 0.71 |
| 0.12 | 0.51 | 0.37 | 0.43 |
| 0.23 | 0.50 | 1.37 | 0.29 |
| 0.81 | 0.12 | 2.56 | 0.43 |
| | Mean ± | SEM | |
| 4.315 ± 3.931 N=4 | 0.5300 ± 0.1782 N=4 | 1.335 ± 0.4583 N=4 | 0.4650 ± 0.08808 N=4 |
| **P>0.05** | | **P=0.045** | |

These results show that serum and mucosal IgG responses in animals primed IN and boosted IM are more robust than animals immunized by IM alone. In addition, IN priming and IM boosting with VEE/SIN replicon particles resulted in high titers than IN priming with SIN and subsequent IM boosting with VEE/SIN particles.

### 3.B. Study Design #2: IgA

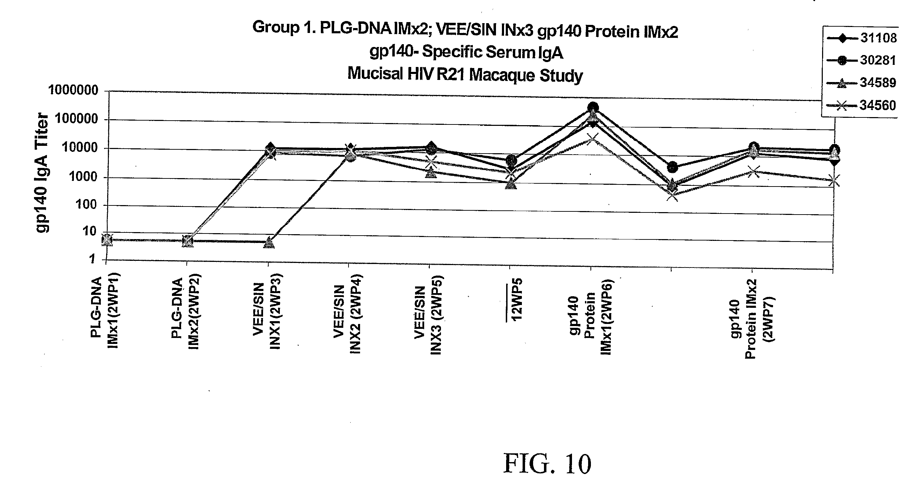

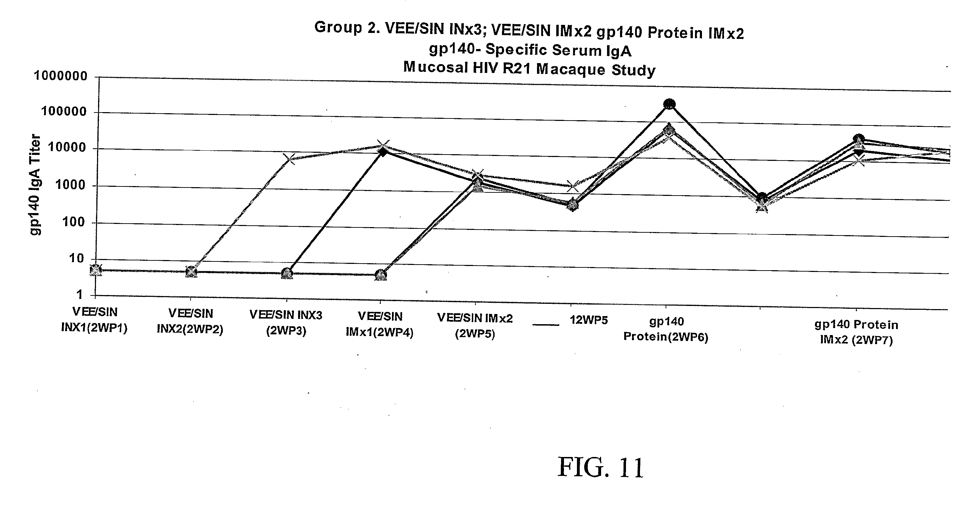

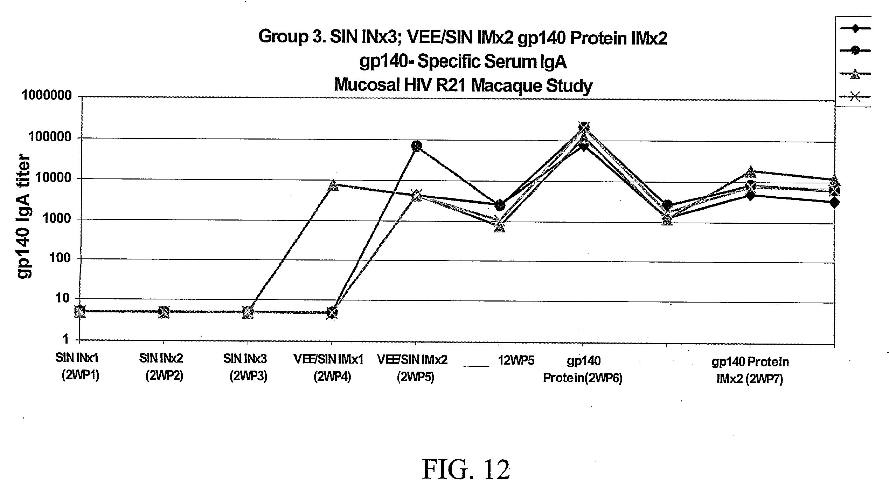

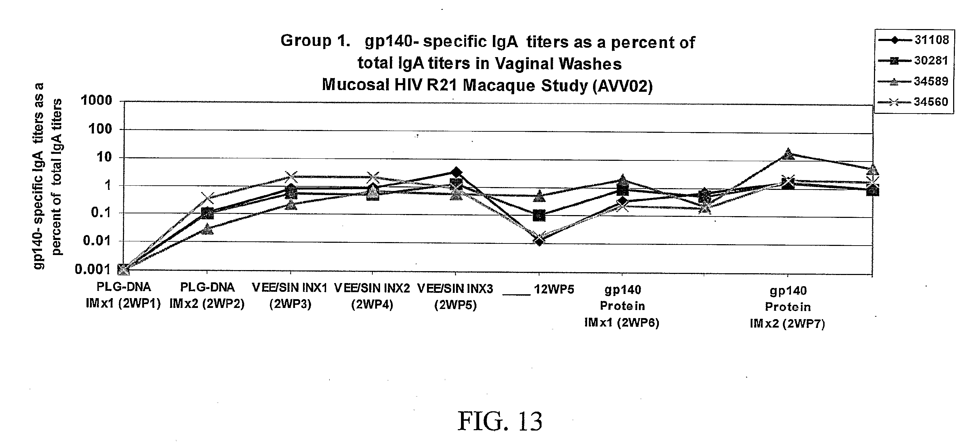

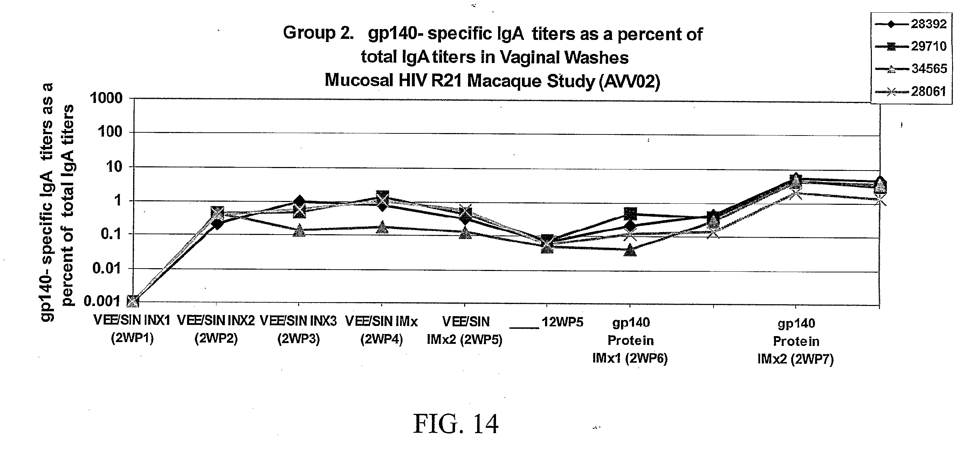

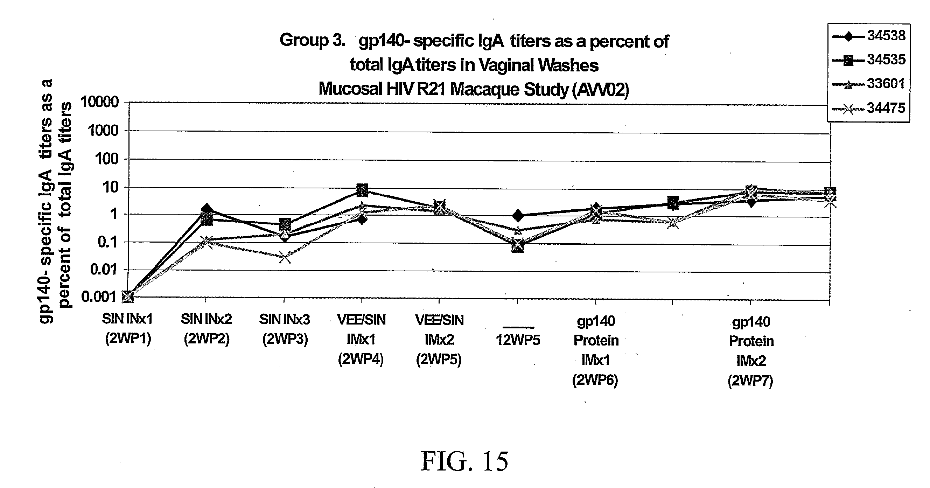

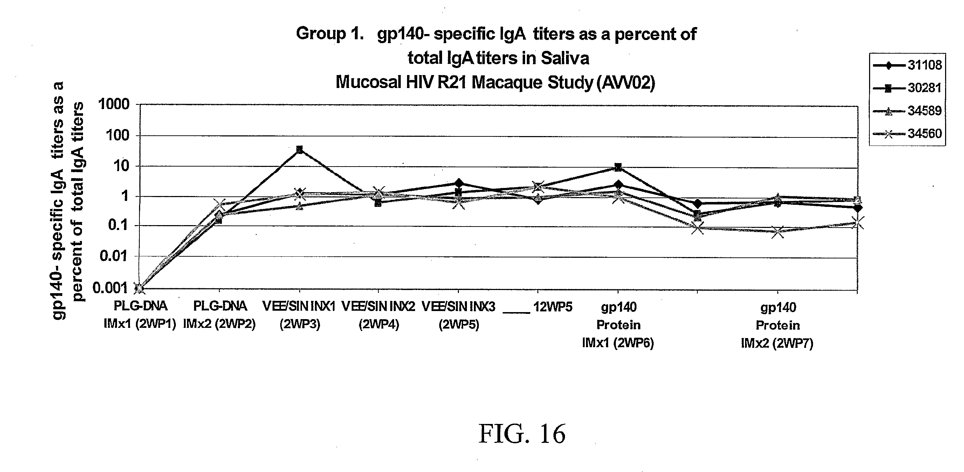

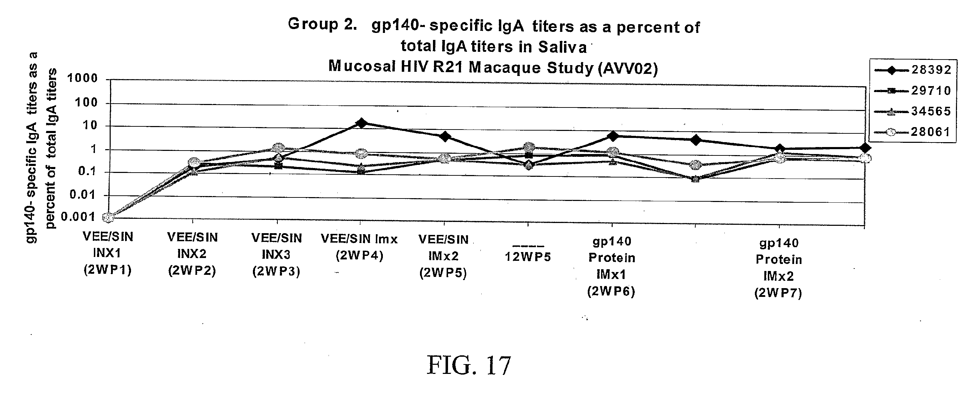

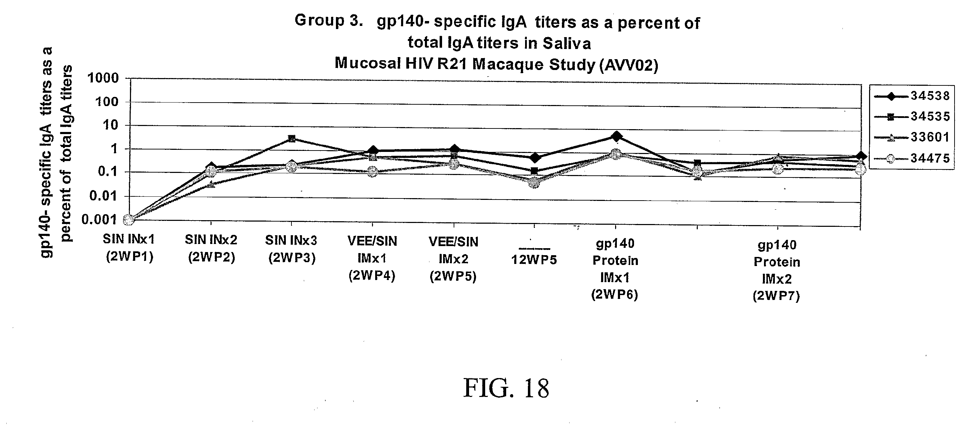

In addition, IgA titers from the groups receiving additional protein boost was also determined from serum (FIGs. 10, 11, 12), vaginal washes (Figs. 13, 14, 15) and saliva (FIGs. 16, 17, 18).

Anti-gp140 serum and vaginal IgA responses were significantly enhanced after intra-muscular boosting immunizations with o-gp140 protein in MF59 following priming IN/IM immunizations with replicon particles. However, anti-gp140 saliva IgA responses were not enhanced after IM boosting immunizations with o-gp140 protein in MF59 following priming with IN/IM immunizations with replicon particles.

### 4. Comparison of ex vivo infection of PBMC with VEE, SIN and VEE/SIN Infected APC Characterization

Because VEE/SIN and SIN have the same envelope glycoprotein structure, we wished to preclude the possibility of their ability to infect target cells as a mechanism for enhanced immunogenicity of VEE/SIN vs. SIN. Thus, to determine the ability of the replicon particles to infect various cell populations, PBMC from naïve macaques were infected *in vitro* with VEE, VEE/SIN or SIN replicon particles expressing a green fluorescent protein (GFP) reporter.

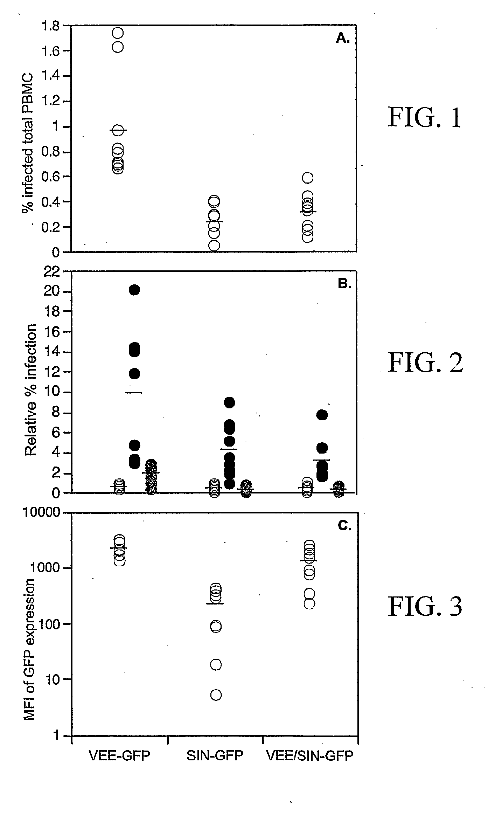

Although the VEE replicon particles infected a higher percentage of cells compared to VEE/SIN (VEE vs. VEE/SIN, p<0.029) or SIN (VEE vs. SIN, p<0.00005), the VEE/SIN and SIN replicon particles infected a similar percentage of cells (Figure 1). Furthermore, as shown in Figure 2, the CD14+ monocyte lineage cells were the predominant targets of the replicon particles. The VEE replicon particles infected the CD1 1b+monocyte population at a relatively low level. The CD20+ B cell population was not infected by any of the replicon particles.

These data show that the VEE/SIN and SIN replicon particles infect the same percentage of cell and their primary targets in PBMC are the CD14+ monocyte lineage cells. Thus, the enhanced immunogenicity of VEE/SIN vs. SIN is likely not due to differences in their ability to infect their target cell population.

### 5. Protein Expression

As the same percentage of cells got infected with VEE/SIN and SIN, it was also determined whether a higher expression of the encoded protein could explain the better immunogenicity of VEE/SIN vs. SIN.

Following *ex vivo* infection of PBMC with VEE/SIN, SIN and VEE expressing GFP, VEE/SIN expressed significantly higher levels of GFP compared to SIN (p<0.002) (Figure 3).

These data indicate that the better immunogenicity of VEE/SIN vs. SIN may be due to the higher intensity of expression of the gene of interest in the infected target cells.

Thus, the experiments described herein demonstrate that the mucosal priming and parenteral boosting immunizations stimulate immune responses in a subject.
Further embodiments of the description are described below in the following numbered paragraphs:
1. Use of a first immunogenic composition comprising one or more alphavirus replicon particles and a second immunogenic composition comprising one or more alphavirus replicon particles in the manufacture of a medicament for mucosal administration of the first immunogenic composition and systemic administration of the second immunogenic composition to thereby generate an immune response in a subject, wherein the alphavirus replicon particles of the first and second immunogenic compositions comprise at least one polynucleotide encoding an antigen.
2. Use of a first immunogenic composition comprising one or more alphavirus replicon particles and a second immunogenic composition comprising one or more alphavirus replicon particles in the manufacture of a medicament for generating an immune response in a subject wherein the first immunogenic composition is administered mucosally and the second immunogenic composition is separately or sequentially administered mucosally, wherein the alphavirus replicon particles of the first and second immunogenic compositions comprise at least one polynucleotide encoding an antigen.
3. Use of a first immunogenic composition comprising one or more alphavirus replicon particles in the manufacture of a medicament for mucosal administration to generate an immune response in a subject wherein the subject will subsequently be systemically administered a second immunogenic composition comprising one or more alphavirus replicon particles, wherein the alphavirus replicon particles of the first and second immunogenic compositions comprise at least one polynucleotide encoding an antigen.
4. Use of a second immunogenic composition comprising one or more alphavirus replicon particles in the manufacture of a medicament for systemic administration to generate an immune response in a subject wherein the subject has already been administered a first immunogenic composition comprising one or more alphavirus replicon particles, wherein the alphavirus replicon particles of the first and second immunogenic compositions comprise at least one polynucleotide encoding an antigen.
5. A product comprising as a combined preparation:
   (a) a first immunogenic composition for mucosal administration which comprises one or more alphavirus replicon particles, wherein said alphavirus replicon particles comprise at least one polynucleotide encoding an antigen; and
   (b) a second immunogenic composition for systemic administration which comprises one or more alphavirus replicon particles, wherein said alphavirus replicon particles comprise at least one polynucleotide encoding an antigen
      for separate or sequential use in generating an immune response in a subject.
6. A method of generating an immune response in a subject, comprising.
   (a) mucosally administering to the subject a first immunogenic composition comprising one or more alphavirus replicon particles, wherein said alphavirus replicon particles comprise at least one polynucleotide encoding an antigen; and
   (b) systemically administering to the subject a second immunogenic composition comprising one or more alphavirus replicon particles, wherein said alphavirus replicon particles comprise at least one polynucleotide encoding an antigen, thereby inducing an immune response in the subject.
7. The use, product or method of any of the preceding embodiments wherein the mucosal administration is intranasal, intrarectal or intravaginal.
8. The use, product or method of any of the preceding embodiments wherein the systemic administration is intramuscular.
9. The use, product or method of any of the preceding embodiments wherein the subject is administered the first immunogenic composition at least two times.
10. The use, product or method of any one of embodiments 1 to 8 wherein the subject is administered the first immunogenic composition at least three times.
11. The use, product or method of any one of the preceding embodiments wherein the subject is administered the second immunogenic composition at least two times.
12. The use, product or method of any one of the preceding embodiments wherein at least one alphavirus replicon particle is derived from an alphavirus selected from the group consisting of: Sindbis (SIN), Venezuelan equine encephalitis (VEE), and Semliki Forest virus (SFV).
13. The use, product or method of any one of the preceding embodiments wherein at least one alphavirus replicon particle is a chimeric VEE/SIN replicon particle.
14. The use, product or method of any one of the preceding embodiments wherein at least one antigen is selected from the group consisting of: a viral antigen, a bacterial antigen and a tumor antigen.
15. The use, product or method of embodiment 14, wherein the viral antigen is derived from a virus selected from the group consisting of: an influenza virus, a respiratory syncytial virus (RSV), a parainfluenza virus (PIV), a hepatitis C virus (HCV), a human immunodeficiency virus (HIV), a herpes simplex virus (HSV), a human papilloma virus (HPV), and a hepatitis B virus (HBV).
16. The use, product or method of any one of the preceding embodiments wherein the first and second immunogenic compositions comprise the same antigen(s).
17. The use, product or method of any one of embodiments 1 to 15 wherein the first and second immunogenic compositions comprise different antigen(s).
18. The use, product or method of embodiment 17 wherein the different antigen(s) are derived from the same pathogen.
19. The use, product or method of embodiment 17 wherein the different antigen(s) are derived from different pathogens.
20. The use, product or method of any one of the preceding embodiments wherein the first and/or second immunogenic compositions further comprise(s) an additional delivery vehicle.
21. The use, product or method of any one of the preceding embodiments wherein the first and/or second immunogenic compositions further comprise(s) an adjuvant.
22. The use, product or method of any one of the preceding embodiments wherein the first and/or second immunogenic compositions further comprise(s) one or more polypeptide antigens.
23. The use, product or method any one of the preceding embodiments wherein the immune response is selected from the group consisting of: a systemic immune response, a mucosal immune response, and both a systemic and mucosal immune response.
24. The use, product or method of any one of embodiments 1, 2 and 5 to 23, wherein step the first immunogenic composition is administered before the second immunogenic composition.
25. The use, product or method of any one of embodiments 1, 2 and 5 to 23, wherein the second immunogenic composition is administered prior to the first immunogenic composition.
26. The use, product or method any one of the preceding embodiments, further comprising one or more polypeptide antigens or one or more polynucleotides encoding one or more antigens for administration to the subject.
27. The use, product or method embodiment 26, wherein the polynucleotides are administered via alphavirus replicon vectors, poxvirus replicon particles, poxvirus replicon vectors, adenovirus replicon particles, adenovirus replicon vectors or combinations of any of the foregoing.

## Claims

1. Use of a first immunogenic composition comprising one or more virus replicon vectors or particles and a second immunogenic composition comprising one or more virus replicon vectors or particles in the manufacture of a medicament for mucosal administration of the first immunogenic composition and systemic administration of the second immunogenic composition to thereby generate an immune response in a subject, wherein the virus replicon vectors or particles of the first and second immunogenic compositions comprise at least one polynucleotide encoding a viral antigen, a bacterial antigen or a tumor antigen and wherein the first immunogenic composition is administered before the second immunogenic composition.

2. Use of a first immunogenic composition comprising one or more virus replicon vectors or particles and a second immunogenic composition comprising one or more virus replicon vectors or particles in the manufacture of a medicament for generating an immune response in a subject wherein the first immunogenic composition is administered mucosally and the second immunogenic composition is separately or sequentially administered systemically, wherein the virus replicon vectors or particles of the first and second immunogenic compositions comprise at least one polynucleotide encoding a viral antigen, a bacterial antigen or a tumor antigen and wherein the first immunogenic composition is administered before the second immunogenic composition.

3. Use of a first immunogenic composition comprising one or more virus replicon particles in the manufacture of a medicament for mucosal administration to generate an immune response in a subject wherein the subject will subsequently be systemically administered a second immunogenic composition comprising one or more virus replicon particles, wherein the virus replicon particles of the first and second immunogenic compositions comprise at least one polynucleotide encoding a viral antigen, a bacterial antigen or a tumor antigen and wherein the first immunogenic composition is administered before the second immunogenic composition.

4. Use of a second immunogenic composition comprising one or more virus replicon particles in the manufacture of a medicament for systemic administration to generate an immune response in a subject wherein the subject has already been administered a first immunogenic composition comprising one or more virus replicon particles, wherein the virus replicon particles of the first and second immunogenic compositions comprise at least one polynucleotide encoding a viral antigen, a bacterial antigen or a tumor antigen and wherein the first immunogenic composition is administered before the second immunogenic composition.

5. A product comprising as a combined preparation:
(a) a first immunogenic composition for mucosal administration which comprises one or more avirus replicon particles, wherein said virus replicon particles comprise at least one polynucleotide encoding a viral antigen, a bacterial antigen or a tumor antigen; and
(b) a second immunogenic composition for systemic administration which comprises one or more virus replicon particles, wherein said virus replicon particles comprise at least one polynucleotide encoding a viral antigen, a bacterial antigen or a tumor antigen,
for separate or sequential use in generating an immune response in a subject wherein the first immunogenic composition is administered before the second immunogenic composition.

6. The use or product of any of claims 1-5, wherein the virus replicon vectors of the first immunogenic composition are poxvirus replicon vectors, alphavirus replicon vectors, or adenovirus replicon vectors and/or wherein the virus replicon vectors of the second immunogenic composition are poxvirus replicon vectors, alphavirus replicon vectors, or adenovirus replicon vectors.

7. The use or product of any of claims 1-5, wherein the virus replicon particles of the first immunogenic composition are poxvirus replicon particles, alphavirus replicon particles, or adenovirus replicon particles and/or wherein the virus replicon particles of the second immunogenic composition are poxvirus replicon particles, alphavirus replicon particles, or adenovirus replicon particles.

8. The use or product of any of the preceding claims wherein the mucosal administration is intranasal, intrarectal or intravaginal.

9. The use or product of any of the preceding claims wherein the systemic administration is intramuscular.

10. The use or product of any of the preceding claims wherein the subject is administered the first immunogenic composition at least two times, and wherein optionally the subject is administered the first immunogenic composition at least three times.

11. The use or product of any one of the preceding claims wherein the subject is administered the second immunogenic composition at least two times.

12. The use or product of any one of the preceding claims wherein the first and second immunogenic compositions comprise the same antigen(s).

13. The use or product of any one of claims 1 to 11 wherein the first and second immunogenic compositions comprise different antigen(s).

14. The use or product of claim 13, wherein:
(i) the different antigen(s) are derived from the same pathogen; or
(ii) the different antigen(s) are derived from different pathogens.

15. The use or product of any one of the preceding claims wherein the first and/or second immunogenic compositions further comprise(s) one or more polypeptide antigens.
